# EUROPEAN PATENT APPLICATION

(11) **EP 4 722 253 A1**
(43) Date of publication of application: **08.04.2026**
(21) Application number: 24811428.2
(22) Date of filing: 23.05.2024
(51) Int. Cl.: C07K 16/46

(54) **CH3 DOMAIN VARIANT OR BISPECIFIC ANTIBODY COMPRISING SAME**

(30) Priority: 24.05.2023 KR 20230066854; 24.05.2023 KR 20230066855
(71) Applicant: Samsung Biologics Co., Ltd., Incheon 21987 (KR)
(72) Inventor: JUNG, Woosuk, Seoul 05698 (KR); KIM, Jungwon, Incheon 21982 (KR); KIM, Migyeom, Incheon 21986 (KR); KIM, Jina, Incheon 21316 (KR); PARK, Juhyun, Incheon 22008 (KR); LEE, Sangho, Goyang-si, Gyeonggi-do 10459 (KR); HONG, Hyebeen, Incheon 22008 (KR); LEE, Soyeon, Incheon 21395 (KR); PARK, Sangyun, Siheung-si, Gyeonggi-do 15011 (KR); YANG, Wooseok, Suwon-si, Gyeonggi-do 16636 (KR); KANG, Seong Ho, Incheon 21986 (KR); SHIN, Gwangsu, Incheon 22008 (KR); KIM, Mina, Incheon 21982 (KR); KIM, Jihoon, Anyang-si, Gyeonggi-do 14075 (KR); YANG, Sujae, Incheon 21998 (KR); OH, Sekyu, Incheon 21982 (KR); WI, Jimin, Incheon 21986 (KR); LEE, Chaeyoung, Incheon 21683 (KR); JEONG, Seonkyeong, Incheon 21948 (KR); CHOI, Hyungseok, Yongin-si, Gyeonggi-do 17000 (KR)
(74) Representative: Mewburn Ellis LLP
(86) International application number: PCT/KR2024/007030
(87) International publication number: WO 2024/242490

(57) **Abstract**

The present invention relates to a heterodimer comprising a CH3 domain variant pair or a bispecific antibody comprising same and, more specifically, to a heterodimer to which a CH3 domain variant pair substituted with an amino acid at a specific position is applied, or a bispecific antibody comprising same.

## Description

### [Technical Field]

The present invention relates to a heterodimer including a CH3 domain variant pair or a bispecific antibody including the same, and more particularly, to a heterodimer including a CH3 domain variant pair with amino acid substitutions at specific positions or a bispecific antibody including the same.

### [Background Art]

As various causes and mechanisms underlying certain indications have recently been elucidated, an approach shifting from a single target to multiple targets has emerged in therapeutic development. Accordingly, in the development of antibody-based therapeutics, numerous studies have been conducted over several decades to confer monospecific antibodies with bispecific or multispecific properties capable of specifically binding to two or more antigen proteins.

A bispecific antibody is an antibody with dual functions, interacting with two different antigens simultaneously. An effective strategy for producing bispecific antibodies is based on the approach of mutating CH3 amino acid residues to form a knob for one half of the antibody and a hole for the remaining half. This knob-into-hole (KiH) technology has been reported to produce approximately 80% heterodimers (Ridgway, Presta et al. 1996).

However, there is still a need for improvement/modification to enhance the bispecificity of currently developed bispecific antibodies.

Against this technical background, the inventors of the present application have ascertained that the bispecificity of antibodies may be enhanced through CH3 domain engineering by constructing a heterodimer including a CH3 domain variant pair including amino acid substitutions at specific positions in addition to the conventional knob-into-hole (KiH) technology, thus culminating in the present invention.

### [Disclosure]

An object of the present invention is to provide a heterodimer including a CH3 domain variant pair.

Another object of the present invention is to provide a fusion protein including the heterodimer.

Still another object of the present invention is to provide a bispecific antibody including the heterodimer including the CH3 domain variant pair.

In order to accomplish the above objects, the present invention provides a heterodimer including a first heavy chain CH3 domain and a second heavy chain CH3 domain,

in which the first heavy chain CH3 domain or the second heavy chain CH3 domain includes at least one amino acid substitution (according to the EU index) at a position selected from the group consisting of:
(1) substitution of the amino acid at position Q347, E356, or E357 with K or R;
(2) substitution of the amino acid at position D399 with W, F, Y, or H;
(3) substitution of the amino acid at position K360, K370, or K439 with Q, N, E, or D; and
(3) substitution of the amino acid at position K409 with A, V, T, or S, and
the first heavy chain CH3 domain and the second heavy chain CH3 domain bind to form a dimer.

The present invention also provides a fusion protein including the heterodimer.

The present invention relates to a bispecific antibody including a first arm that binds to a first antigen including VH1-CHa-Fc1 and VL1-CLb and a second arm that binds to a second antigen including VH2-CH1-Fc2 and VL2-CL,
in which the VH1 and VH2 are heavy chain variable regions including identical or different antigen binding regions,
the VL1 and VL2 are light chain variable regions including identical or different antigen binding regions,
the CHa includes i) an IgG heavy chain constant region or an IgD heavy chain constant region CH1, and an IgG heavy chain constant region CH2 or CH3,
the CLb includes at least one selected from the group consisting of i) CL1 including an IgG light chain constant region λ or κ, and IgG heavy chain constant regions CH1, CH2, and CH3,
the CH1 is an IgG heavy chain constant region CH1, and the CL is an IgG light chain constant region CL,
F_{c1} of the first arm and F_{c2} of the second arm bind to form a heavy chain constant region dimer, and
the bispecific antibody includes a heterodimer including a first heavy chain CH3 domain and a second heavy chain CH3 domain, in which CH3 of the CHa, F_{c1} of the first arm, or F_{c2} of the second arm includes at least one amino acid substitution (according to the EU index) at a position selected from the group consisting of:
   (1) substitution of the amino acid at position Q347, E356, or E357 with K or R;
   (2) substitution of the amino acid at position D399 with W, F, Y, or H;
   (3) substitution of the amino acid at position K360, K370, or K439 with Q, N, E, or D; and
   (3) substitution of the amino acid at position K409 with A, V, T, or S.

### [Brief description of Drawings]

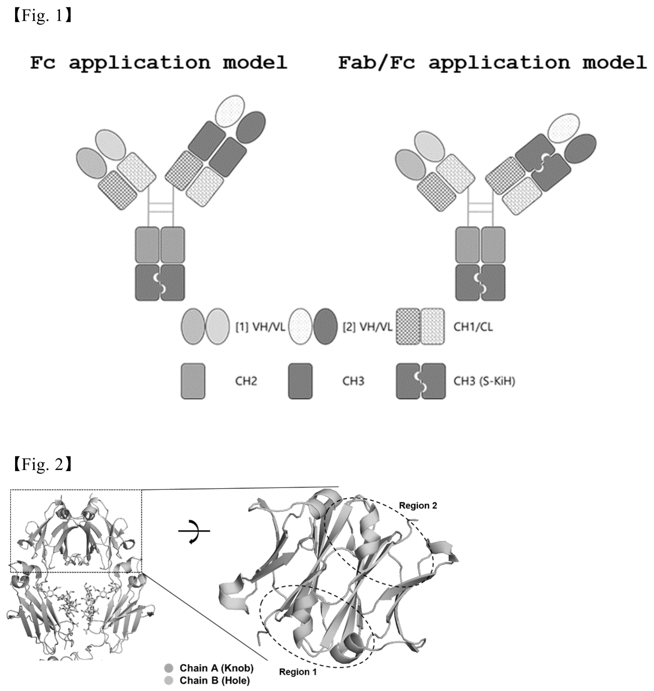

FIG. 1 shows the structure of a bispecific antibody including a heterodimer including a mutation according to the present invention;
FIG. 2 shows the overall structure and mutation target region of the CH3 domain of the bispecific antibody;

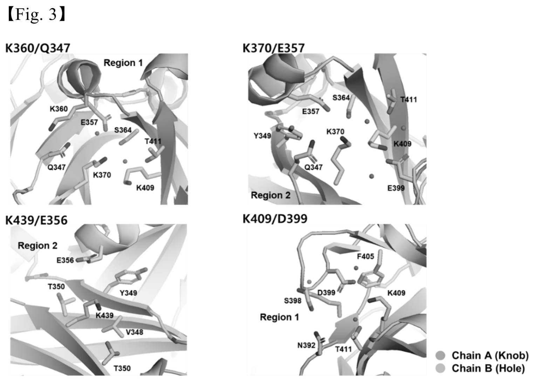

FIG. 3 shows mutation candidate regions;

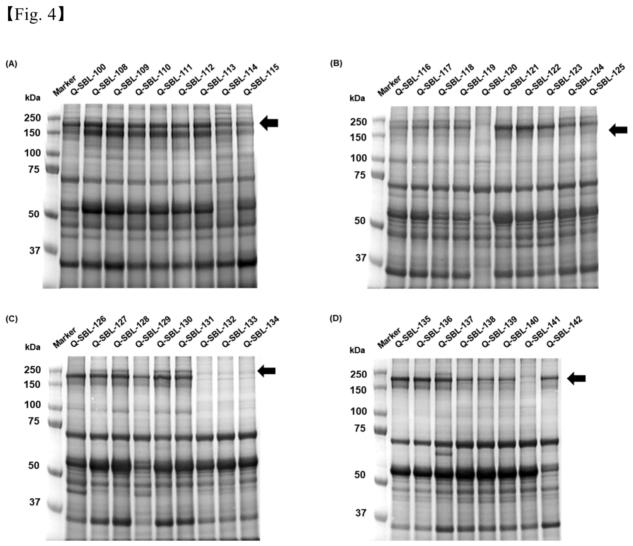

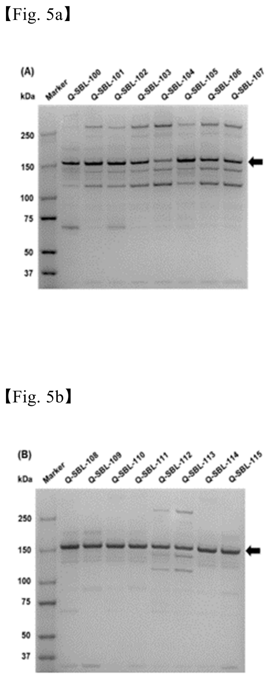

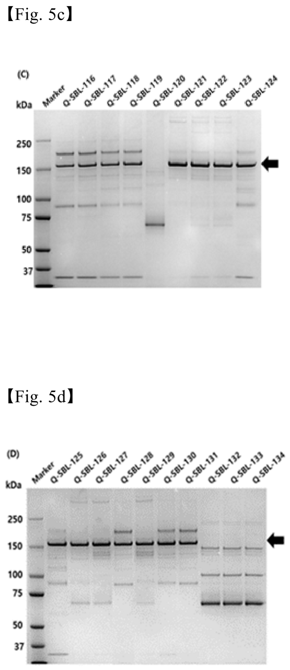

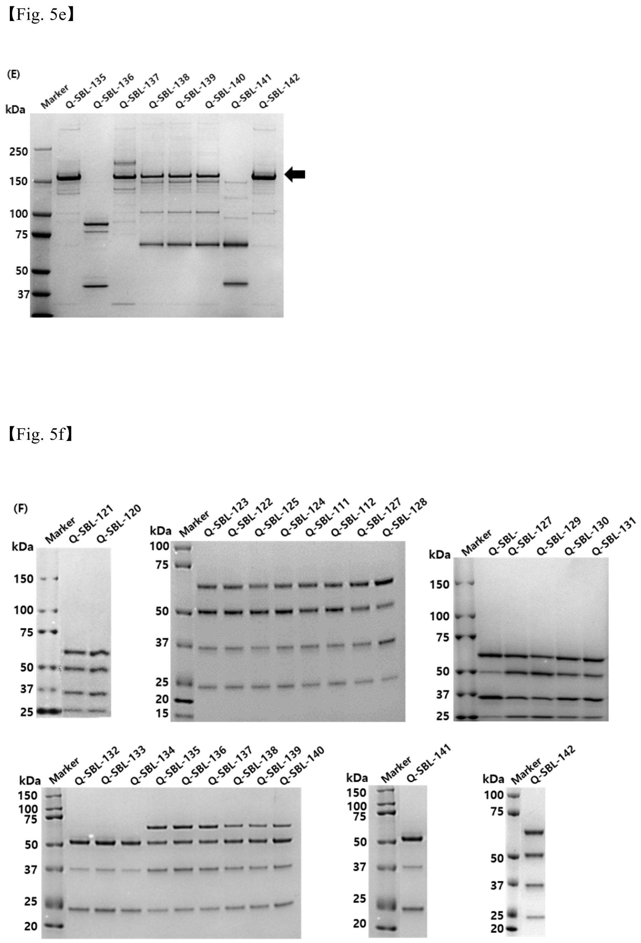

FIG. 4 shows results of expression pattern analysis through non-reducing SDS-PAGE of the filtered supernatant; FIGs. 5a to 5f show results of non-reducing and reducing SDS-PAGE analyses of primary purified products of bispecific antibody candidates;
FIG. 6 shows results of ELISA for single antigen binding including ErbB2 and VEGF binding regions;
FIG. 7 shows results of ELISA for dual antigen binding including ErbB2 and VEGF binding regions; and
FIG. 8 shows results of quantitative analysis of the binding strength between bispecific antibody and ErbB2 expressed on the surface of JIMT-1 cells using FACS.

### [Mode for Invention]

Unless otherwise defined, all technical and scientific terms used herein have the same meanings as typically understood by those skilled in the art to which the present invention belongs. In general, the nomenclature used herein is well known in the art and is typical.

An aspect of the present invention relates to a heterodimer including a first heavy chain CH3 domain and a second heavy chain CH3 domain,
in which the first heavy chain CH3 domain or the second heavy chain CH3 domain includes at least one amino acid substitution (according to the EU index) at a position selected from the group consisting of:
   (1) substitution of the amino acid at position Q347, E356, or E357 with K or R;
   (2) substitution of the amino acid at position D399 with W, F, Y, or H;
   (3) substitution of the amino acid at position K360, K370, or K439 with Q, N, E, or D; and
   (3) substitution of the amino acid at position K409 with A, V, T, or S, and
   the first heavy chain CH3 domain and the second heavy chain CH3 domain bind to form a dimer.

The heterodimer is based on a first heavy chain CH3 domain and a second heavy chain CH3 domain, each of which may be considered a monomer, and "monomer" may mean 1/2 of the heterodimer. The heterodimer may be formed by assembly of monomers. A heterodimer may be formed by inducing an amino acid mutation in each of the monomers and assembling the mutated monomers.

Specifically, knobs-into-holes (KiH) may be formed by including mutations that create steric effects that are favorable for heterodimer formation and unfavorable for homodimer formation. In the heterodimer, each of the monomers, particularly the first heavy chain CH3 domain and the second heavy chain CH3 domain, includes a mutation, for example an amino acid substitution, thereby forming a pair of mutated CH3 domains.

With regard to knobs-into-holes (KiH), the first heavy chain CH3 domain may include a mutation, for example an amino acid substitution, to form a knob, and the second heavy chain CH3 domain may include a mutation, for example an amino acid substitution, to form a hole, thereby forming knobs-into-holes (KiH).

Accordingly, the first heavy chain CH3 domain or the second heavy chain CH3 domain includes at least one amino acid substitution (according to the EU index) at a position selected from the group consisting of:
(1) substitution of the amino acid at position Q347, E356, or E357 with K or R;
(2) substitution of the amino acid at position D399 with W, F, Y, or H;
(3) substitution of the amino acid at position K360, K370, or K439 with Q, N, E, or D; and
(3) substitution of the amino acid at position K409 with A, V, T, or S.

The first heavy chain CH3 domain and the second heavy chain CH3 domain bind to form a dimer.

Another aspect of the present invention relates to a bispecific antibody including a first arm that binds to a first antigen including VH1-CHa-Fc1 and VL1-CLb and a second arm that binds to a second antigen including VH2-CH1-Fc2 and VL2-CL.

Here, the VH1 and VH2 are heavy chain variable regions including identical or different antigen binding regions.

The VL1 and VL2 are light chain variable regions including identical or different antigen binding regions.

The CHa includes i) an IgG heavy chain constant region or an IgD heavy chain constant region CH1, and an IgG heavy chain constant region CH2 or CH3.

The CLb includes at least one selected from the group consisting of i) CL1 including an IgG light chain constant region λ or κ, and IgG heavy chain constant regions CH1, CH2, and CH3.

The CH1 is an IgG heavy chain constant region CH1, and the CL is an IgG light chain constant region CL.

F_{c1} of the first arm and F_{c2} of the second arm bind to form a heavy chain constant region dimer.

The bispecific antibody includes a heterodimer including a first heavy chain CH3 domain and a second heavy chain CH3 domain, in which CH3 of the CHa, F_{c1} of the first arm, or F_{c2} of the second arm includes at least one amino acid substitution (according to the EU index) at a position selected from the group consisting of:
(1) substitution of the amino acid at position Q347, E356, or E357 with K or R;
(2) substitution of the amino acid at position D399 with W, F, Y, or H;
(3) substitution of the amino acid at position K360, K370, or K439 with Q, N, E, or D; and
(3) substitution of the amino acid at position K409 with A, V, T, or S.

The present invention provides a new form of bispecific antibody that is different from the bispecific antibody disclosed in Korean Patent Application Publication No. 2022-0095163, and is capable of increasing the yield of heterodimer formation compared to conventional bispecific antibodies by minimizing mispairing by introducing different mutation pairs into the CH3 domains redundantly present in Fc and Fab.

The heterodimer is based on a first heavy chain CH3 domain and a second heavy chain CH3 domain, each of which may be considered a monomer, and "monomer" may mean 1/2 of the heterodimer. The heterodimer may be formed by assembly of monomers. A heterodimer may be formed by inducing an amino acid mutation in each of the monomers and assembling the mutated monomers.

Specifically, knobs-into-holes (KiH) may be formed by including mutations that create steric effects that are favorable for heterodimer formation and unfavorable for homodimer formation. In the heterodimer, each of the monomers, particularly the first heavy chain CH3 domain and the second heavy chain CH3 domain, includes a mutation, for example an amino acid substitution, thereby forming a pair of mutated CH3 domains.

With regard to knobs-into-holes (KiH), the first heavy chain CH3 domain may include a mutation, for example an amino acid substitution, to form a knob, and the second heavy chain CH3 domain may include a mutation, for example an amino acid substitution, to form a hole, thereby forming knobs-into-holes (KiH).

Accordingly, the first heavy chain CH3 domain or the second heavy chain CH3 domain includes at least one amino acid substitution (according to the EU index) at a position selected from the group consisting of:
(1) substitution of the amino acid at position Q347, E356, or E357 with K or R;
(2) substitution of the amino acid at position D399 with W, F, Y, or H;
(3) substitution of the amino acid at position K360, K370, or K439 with Q, N, E, or D; and
(3) substitution of the amino acid at position K409 with A, V, T, or S.

The first heavy chain CH3 domain and the second heavy chain CH3 domain bind to form a dimer.

The antibody binding to the heterodimer may be of any type, including but not limited to Fab, Fab', F(ab')2, vH, vL, Fv, scFv, scFv2, scFab, or dAb.

"Dual-specific" or "bispecific" refers to the property of a binding protein to specifically bind to two different targets and regulate the activity of the targets. For example, such a bispecific protein may be produced by conjugating monoclonal antibodies or fragments thereof, each of which specifically binds to a target, and the bispecific antibody possesses two distinct antigen binding arms (specificity for two targets) and is monovalent for each antigen binding thereto.

The VH1 and VH2 are heavy chain variable regions including identical or different antigen binding regions. The VL1 and VL2 are light chain variable regions including identical or different antigen binding regions.

"Polypeptide" refers to any polymer chain of amino acids. The terms "peptide" and "protein" may be used interchangeably with the term polypeptide, and likewise refer to a polymer chain of amino acids. "Polypeptide" includes natural or synthetic proteins, protein fragments, and polypeptide analogues of protein sequences. A polypeptide may be a monomer or a polymer.

In relation to interactions of antibodies, polypeptides, proteins, or peptides, "specific binding" or "specifically binds" refers to an interaction that occurs depending on the presence of a particular structure on a chemical species (e.g., an antigenic determinant or epitope). For example, antibodies generally recognize and bind to specific protein structures rather than proteins. If the antibody is specific for epitope "A", then the presence of a molecule containing epitope A (or free unlabeled A) in a reaction involving labeled "A" and the antibody will decrease the amount of labeled A bound to the antibody.

An antibody is any immunoglobulin (Ig) molecule composed of four polypeptide chains, namely two heavy chains (H) and two light chains (L), or any functional fragment, mutant, variant, or derivative of such an Ig molecule having the essential epitope binding characteristics. Specific examples of such mutants, variants, or derivatives are discussed below, but are not limited thereto.

"Monoclonal antibody" refers to an antibody obtained from a substantially homogeneous population of antibodies, in which individual antibodies within the population are identical except for naturally occurring mutations that may be present in small amounts. Monoclonal antibodies are highly specific, being directed against a single antigenic site. In contrast to typical (polyclonal) antibody preparations, which include different antibodies directed against different determinants (epitopes), each monoclonal antibody is directed against a single determinant on the antigen.

"Epitope" refers to a protein determinant to which an antibody may specifically bind. Epitopes are typically composed of chemically active surface molecules, such as amino acids or sugar side chains, and generally have not only specific three-dimensional structural features but also distinct charge characteristics. Conformational epitopes and linear epitopes are distinguished in that binding to the former is lost in the presence of a denaturing solvent, but binding to the latter is not.

A "humanized" form of a non-human (e.g., murine) antibody is a chimeric antibody that contains a minimum sequence derived from a non-human immunoglobulin. In most cases, a humanized antibody refers to a human immunoglobulin (recipient antibody) in which residues in the hypervariable regions of the recipient are replaced with residues in the hypervariable regions of a non-human species (donor antibody), such as a mouse, rat, rabbit, or non-human primate, that possesses the desired specificity, affinity, and functionality.

"Human antibody" refers to a molecule derived from human immunoglobulin, in which the entire amino acid sequence constituting the antibody, including complementarity determining regions and framework regions, is composed of human immunoglobulin.

In a complete antibody, each heavy chain is composed of a heavy chain variable region (designated HCVR or VH) and a heavy chain constant region. The heavy chain constant region is composed of three domains, CH1, CH2, and CH3. Each light chain is composed of a light chain variable region and a light chain constant region. The light chain constant region is composed of one domain, CL. The VH and VL regions may be further divided into hypervariable regions called complementarity determining regions (CDRs), interspersed with more conserved regions called framework regions (FRs). "Variable region" refers to the light and heavy chain portions of an antibody molecule including the amino acid sequences of the complementarity determining regions (CDRs; i.e., CDR1, CDR2, and CDR3) and the framework regions (FR). VH refers to the variable region of the heavy chain. VL refers to the variable region of the light chain.

"Complementarity determining regions" (CDRs; i.e., CDR1, CDR2, and CDR3) refer to amino acid residues in the antibody variable region that are necessary for antigen binding. Each variable region typically has three CDR regions, identified as CDR1, CDR2, and CDR3. "Framework regions" (FR) are variable region residues other than CDR residues. Each variable region typically has four FRs, identified as FR1, FR2, FR3, and FR4. They are arranged in the following order from amino terminus to carboxyl terminus: FR1, CDR1, FR2, CDR2, FR3, CDR3, and FR4.

The Fab fragment has a structure with variable regions of the light and heavy chains, constant regions of the light chain, and the first constant region (CH1) of the heavy chain, and has one antigen binding site. The Fab' fragment differs from the Fab in that it has a hinge region containing one or more cysteine residues at the C-terminus of the heavy chain CH1 domain. F(ab')2 is produced when cysteine residues in the hinge region of Fab' form a disulfide bond. Fv is the smallest antibody fragment having only a heavy chain variable region and a light chain variable region. Two-chain Fv refers to a structure in which the heavy chain variable region and the light chain variable region are connected through non-covalent bonding, and single-chain Fv (scFv) generally refers to a structure in which the heavy chain variable region and the light chain variable region are linked by a peptide linker through covalent bonding or are directly connected at C-terminus, thereby forming a dimer-like structure similar to that of the two-chain Fv. Such antibody fragments may be obtained using proteolytic enzymes (e.g., by restriction digestion of the whole antibody with papain to obtain Fab, and by digestion with pepsin to obtain F(ab')2), and may also be constructed through recombinant DNA technology.

The antibody includes at least one "antigen binding site" as an antibody fragment that has specific binding ability to an antigen, and may specifically bind to another antigen and may thus be dual-specific or bispecific, or multispecific. In the present invention, the antigen binding site is included in the heavy chain variable region of VH1 or VH2 and the light chain variable region of VL1 or VL2, each including an antigen binding region, which is identical or different.

According to the present invention, the antibody does not significantly deviate from the IgG-like structure. A structure that significantly disrupts the IgG framework is likely to greatly increase protein instability.

Moreover, to drastically reduce the frequency of light chain mispairing, additional constant regions of the immunoglobulin structure were introduced. By introducing a known heavy chain constant region domain into one of the two Fab arms, the formation of mispaired light chains was suppressed.

The bispecific antibody may have two Fab arms that bind to respective targets, in which the left arm (second arm) has no structural modification and the right arm (first arm) is additionally engineered to include an Ig constant domain (CH3), and also, IgG1 CH1 as well as other types of CH1 instead of IgG1 CH1 may be introduced, and the heterodimeric configuration of the heavy chains may be achieved by introducing a knob-in-hole structure.

The CHa may include i) an IgG heavy chain constant region or an IgD heavy chain constant region CH1, and an IgG heavy chain constant region CH2 or CH3.

For example, CHa in the first arm may include, in order from N-terminus to C-terminus, a heavy chain constant region CH1 derived from IgG1, IgG2, IgG3, IgG4, IgD, or IgM, and CH3 derived from IgG1, IgG2, IgG3, or IgG4. For example, CHa in the first arm may include, in order from N-terminus to C-terminus, CH3 derived from IgG1, IgG2, IgG3, or IgG4, and a heavy chain constant region CH1 derived from IgG1, IgG2, IgG3, IgG4, IgD, or IgM.

The CLb includes at least one selected from the group consisting of i) CL1 including an IgG light chain constant region λ or κ, and IgG heavy chain constant regions CH1, CH2, and CH3.

The CLb may include at least one selected from the group consisting of CL1 including a light chain constant region λ or κ derived from IgG1, IgG2, IgG3, IgG4, IgD, or IgM, and heavy chain constant regions CH1, CH2, and CH3 derived from IgG1, IgG2, IgG3, or IgG4. For example, CLb in the first arm may include, in order from N-terminus to C-terminus, CL1 including a light chain constant region λ or κ derived from IgG1, IgG2, IgG3, IgG4, IgD, or IgM, and a heavy chain constant region CH3 derived from IgG1, IgG2, IgG3, or IgG4. For example, CLb in the first arm may include, in order from N-terminus to C-terminus, CH3 derived from IgG1, IgG2, IgG3, or IgG4, and CL1 including a light chain constant region λ or κ derived from IgG1, IgG2, IgG3, IgG4, IgD, or IgM.

The CH1 is an IgG heavy chain constant region CH1, and the CL is an IgG light chain constant region CL. Each of the CH1 and CL may be derived from IgG1, IgG2, IgG3, IgG4, or IgD. The CH1 and CL may have a structure similar to a Fab fragment through non-covalent interactions.

"Fc region" is used to define the C-terminal region of an immunoglobulin heavy chain that may be generated by papain digestion of an intact antibody. The Fc region may be a native Fc region or a variant Fc region. The Fc region of an immunoglobulin typically contains two constant domains, a CH2 domain and a CH3 domain, and optionally a CH4 domain.

In one embodiment, each of the CHa and CLb may include CH3. Specifically, each of the CHa and CLb may include CH3 derived from IgG1, IgG2, IgG3, or IgG4. The CHa and CLb may form a dimer through covalent or non-covalent interactions.

In some cases, the CH3 domain and the CH1 domain may be connected by a linker. The linker may be a peptide linker and may include about 5-25 aa residues, particularly about 5-10 aa residues. For example, hydrophilic amino acids such as, but not limited to, glycine and/or serine may be included.

Specifically, the linker may include, for example, a glycine linker (G, Gly)p (in which p is 1 to 10), or a GS linker (GnS)m (in which n and m are each 1 to 10) to impart structural flexibility. Specifically, the linker may include GGGGS or (GGGGS)2, or may include glycine of 5-10 aa where p is 5-10 in (G, Gly)p.

The first and second arms may be connected via a hinge. The first and second arms may be connected via a hinge formed by including at least one sequence selected from the group consisting of:
DKTHTCPPCP;
EPKSSDKTHTCPPCP; and
ESKYGPPCPPCP.

Each of the F_{c1} of the first arm and the F_{c2} of the second arm includes CH2 and CH3 monomers of the heavy chain constant region. The monomer refers to one of the domains constituting a dimer formed through two CH2-CH3 constant domains having identical amino acid sequences in the heavy chain constant region Fc. The F_{c1} of the first arm and the F_{c2} of the second arm bind to form a heavy chain constant region dimer.

The dimer may include a homodimer formed by binding between CH3 constant domains having identical amino acid sequences or a heterodimer formed by binding between CH3 constant domains having different amino acid sequences.

"Amino acid substitution" or "substitution" may refer to the replacement of an amino acid at a specific position within the amino acid sequence of a CH3 domain. In particular, in some embodiments, the substitution refers to the inclusion of amino acids that do not naturally occur at a given position or that differ from the amino acid sequence occurring naturally at that position.

The CH3 domain is derived from an immunoglobulin (Ig) domain, which is a region of an immunoglobulin having a tertiary structure, and may be derived from an immunoglobulin selected from the group consisting of IgG, IgM, IgA, IgD, and IgE, but may specifically be derived from IgG. The IgG may be human IgG.

In the context of human IgG, the "CH" domain is as follows: "CH1" means positions 118 to 220 according to the EU index, "CH2" means positions 237 to 340 according to the EU index, and "CH3" means positions 341 to 447 according to the EU index.

The sequence of the first domain in the CH3 domain at positions 341 to 447 according to the EU index is as follows:

GQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLS
PGK (SEQ ID NO: 1).

The sequence of the second domain in the CH3 domain at positions 341 to 447 according to the EU index is as follows:

GQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLS
PGK (SEQ ID NO: 2).

Below is a description based on the CH3 domain at positions 341 to 447 of IgG according to the EU index.

Specifically, the amino acid at position Q347, E356, or E357 in the first heavy chain CH3 domain may be substituted with K or R, or the amino acid at position D399 may be substituted with W, F, Y, or H.

In one embodiment, the first heavy chain CH3 domain may include at least one selected from the group consisting of Q347K, Q347R, E357K, E357R, E356K, E356R, D399W, D399F, D399Y, and D399H. Specifically, the first heavy chain CH3 domain may include at least one selected from the group consisting of Q347K, E357K, E356K, D399W, D399F, D399Y, and D399H.

Additionally, the amino acid at position K360, K370, or K439 in the second heavy chain CH3 domain may be substituted with Q, N, E, or D, or the amino acid at position K409 may be substituted with A, V, T, or S.

In one embodiment, the second heavy chain CH3 domain may include at least one selected from the group consisting of K360Q, K360N, K360E, K360D, K370E, K370D, K370N, K370Q, K439E, K439D, K439N, K439Q, K409A, K409V, K409T, and K409S. Specifically, the second heavy chain CH3 domain may include at least one selected from the group consisting of K360Q, K360N, K360E, K360D, K370E, K439E, K439D, K409A, K409T, and K409S.

In one embodiment, the substitution may include at least one, at least two, at least three, or all four selected from among the following, including:
Q347K or Q347R in the first heavy chain CH3 domain, and K360Q, K360N, K360E, or K360D in the second heavy chain CH3 domain;
E357K or E357R in the first heavy chain CH3 domain, and K370Q, K370N, K370E, or K370D in the second heavy chain CH3 domain;
E356K or E356R in the first heavy chain CH3 domain, and K439Q, K439N, K439E, or K439D in the second heavy chain CH3 domain; and
D399W, D399F, D399Y, or D399H in the first heavy chain CH3 domain, and K409A, K409T, K409V, or K409S in the second heavy chain CH3 domain.

In one embodiment, the substitution may include at least one, at least two, at least three, or all four selected from among the following, including:
Q347K in the first heavy chain CH3 domain, and K360Q, K360N, K360E, or K360D in the second heavy chain CH3 domain;
E357K in the first heavy chain CH3 domain, and K370E in the second heavy chain CH3 domain;
E356K in the first heavy chain CH3 domain, and K439E or K439Q in the second heavy chain CH3 domain; and
D399W, D399F, D399Y, or D399H in the first heavy chain CH3 domain, and K409A, K409T, or K409S in the second heavy chain CH3 domain.

In a specific embodiment according to the present invention, the first heavy chain CH3 domain may include at least one selected from the group consisting of Q347K, E357K, E356K, D399W, D399F, D399Y, and D399H, and the second heavy chain CH3 domain may include at least one selected from the group consisting of K360Q, K360N, K360E, K360D, K370E, K439E, K439D, K409A, K409T, and K409S.

In a specific embodiment according to the present invention, the substitution may include at least one combination selected from among the following, including:
Q347K in the first heavy chain CH3 domain and K360Q in the second heavy chain CH3 domain, and E357K in the first heavy chain CH3 domain and K370E in the second heavy chain CH3 domain;
Q347K in the first heavy chain CH3 domain and K360Q in the second heavy chain CH3 domain, and E356K in the first heavy chain CH3 domain and K439E in the second heavy chain CH3 domain;
E357K in the first heavy chain CH3 domain and K370E in the second heavy chain CH3 domain, and E356K in the first heavy chain CH3 domain and K439E in the second heavy chain CH3 domain;
Q347K in the first heavy chain CH3 domain and K360Q in the second heavy chain CH3 domain, and D399W in the first heavy chain CH3 domain and K409A in the second heavy chain CH3 domain;
E357K in the first heavy chain CH3 domain and K370E in the second heavy chain CH3 domain, and D399W in the first heavy chain CH3 domain and K409A in the second heavy chain CH3 domain;
E356K in the first heavy chain CH3 domain and K439E in the second heavy chain CH3 domain, and D399W in the first heavy chain CH3 domain and K409A in the second heavy chain CH3 domain;
Q347K in the first heavy chain CH3 domain and K360Q in the second heavy chain CH3 domain, E357K in the first heavy chain CH3 domain and K370E in the second heavy chain CH3 domain, and E356K in the first heavy chain CH3 domain and K439E in the second heavy chain CH3 domain;
E357K in the first heavy chain CH3 domain and K370E in the second heavy chain CH3 domain, D399W in the first heavy chain CH3 domain and K409A in the second heavy chain CH3 domain, and E356K in the first heavy chain CH3 domain and K439E in the second heavy chain CH3 domain;
Q347K in the first heavy chain CH3 domain and K360Q in the second heavy chain CH3 domain, D399W in the first heavy chain CH3 domain and K409A in the second heavy chain CH3 domain, and E356K in the first heavy chain CH3 domain and K439E in the second heavy chain CH3 domain; and
Q347K in the first heavy chain CH3 domain and K360Q in the second heavy chain CH3 domain, E357K in the first heavy chain CH3 domain and K370E in the second heavy chain CH3 domain, D399W in the first heavy chain CH3 domain and K409A in the second heavy chain CH3 domain, and E356K in the first heavy chain CH3 domain and K439E in the second heavy chain CH3 domain.

In some cases, the first heavy chain CH3 domain or the second heavy chain CH3 domain may further include at least one selected from the group consisting of Y349C, S354C, T366S, T366W, L368A, and Y407V.

In one embodiment, one of the first heavy chain CH3 domain or the second heavy chain CH3 domain may further include at least one selected from the group consisting of Y349C, S354C, T366S, T366W, L368A, and Y407V, and the remaining one of the first heavy chain CH3 domain or the second heavy chain CH3 domain may further include at least one selected from the group consisting of S354C, Y349C, T366W, T366S, L368A, and Y407V.

Specifically, the following residue mutations according to the present invention may be included.

| **No.** | **First domain** | **Second domain** |
|---|---|---|
| 1 | Q347K, S354C, T366W | Y349C, K360Q, T366S, L368A, Y407V |
| 2 | Q347K, S354C, T366W | Y349C, K360N, T366S, L368A, Y407V |
| 3 | Q347K, S354C, T366W | Y349C, K360E, T366S, L368A, Y407V |
| 4 | Q347K, S354C, T366W | Y349C, K360D, T366S, L368A, Y407V |
| 5 | Q347R, S354C, T366W | Y349C, K360Q, T366S, L368A, Y407V |
| 6 | Q347R, S354C, T366W | Y349C, K360N, T366S, L368A, Y407V |
| 7 | Q347R, S354C, T366W | Y349C, K360E, T366S, L368A, Y407V |
| 8 | Q347R, S354C, T366W | Y349C, K360D, T366S, L368A, Y407V |
| 9 | S354C, E357K, T366W | Y349C, T366S, L368A, K370E, Y407V |
| 10 | S354C, E357K, T366W | Y349C, T366S, L368A, K370D, Y407V |
| 11 | S354C, E357K, T366W | Y349C, T366S, L368A, K370N, Y407V |
| 12 | S354C, E357K, T366W | Y349C, T366S, L368A, K370Q, Y407V |
| 13 | S354C, E357R, T366W | Y349C, T366S, L368A, K370E, Y407V |
| 14 | S354C, E357R, T366W | Y349C, T366S, L368A, K370D, Y407V |
| 15 | S354C, E357R, T366W | Y349C, T366S, L368A, K370N, Y407V |
| 16 | S354C, E357R, T366W | Y349C, T366S, L368A, K370Q, Y407V |
| 17 | S354C, E356K, T366W | Y349C, T366S, L368A, Y407V, K439E |
| 18 | S354C, E356K, T366W | Y349C, T366S, L368A, Y407V, K439D |
| 19 | S354C, E356K, T366W | Y349C, T366S, L368A, Y407V, K439N |
| 20 | S354C, E356K, T366W | Y349C, T366S, L368A, Y407V, K439Q |
| 21 | S354C, E356R, T366W | Y349C, T366S, L368A, Y407V, K439E |
| 22 | S354C, E356R, T366W | Y349C, T366S, L368A, Y407V, K439D |
| 23 | S354C, E356R, T366W | Y349C, T366S, L368A, Y407V, K439N |
| 24 | S354C, E356R, T366W | Y349C, T366S, L368A, Y407V, K439Q |
| 25 | S354C, T366W, D399W | Y349C, T366S, L368A, Y407V, K409A |
| 26 | S354C, T366W, D399F | Y349C, T366S, L368A, Y407V, K409V |
| 27 | S354C, T366W, D399F | Y349C, T366S, L368A, Y407V, K409T |
| 28 | S354C, T366W, D399F | Y349C, T366S, L368A, Y407V, K409S |
| 29 | S354C, T366W, D399Y | Y349C, T366S, L368A, Y407V, K409V |
| 30 | S354C, T366W, D399Y | Y349C, T366S, L368A, Y407V, K409T |
| 31 | S354C, T366W, D399Y | Y349C, T366S, L368A, Y407V, K409S |
| 32 | S354C, T366W, D399H | Y349C, T366S, L368A, Y407V, K409V |
| 33 | S354C, T366W, D399H | Y349C, T366S, L368A, Y407V, K409T |
| 34 | S354C, T366W, D399H | Y349C, T366S, L368A, Y407V, K409S |
| 35 | Q347K, S354C, E357K, T366W | Y349C, K360Q, T366S, L368A K370E, Y407V |
| 36 | Q347K, S354C, E356K, T366W | Y349C, K360Q, T366S, L368A, Y407V, K439E |
| 37 | S354C, E357K, E356K, T366W | Y349C, T366S, L368A, K370E, Y407V, K439E |
| 38 | Q347K, S354C, T366W, D399W | Y349C, K360Q, T366S, L368A, Y407V, K409A |
| 39 | S354C, E357K, T366W, D399W | Y349C, T366S, L368A, K370E, Y407V, K409A |
| 40 | S354C, E356K, T366W, D399W | Y349C, T366S, L368A, Y407V, K409A, K439E |
| 41 | Q347K, S354C, E356K, E357K, T366W | Y349C, K360Q, T366S, L368A, K370E, Y407V, K439E |
| 42 | S354C, E356K, E357K, T366W, D399W | Y349C, T366S, L368A, K370E, Y407V, K409A, K439E |
| 43 | Q347K, S354C, E356K, T366W, D399W | Y349C, K360Q, T366S, L368A, Y407V, K409A, K439E |
| 44 | Q347K, S354C, E357K, T366W, D399W | Y349C, K360Q, T366S, L368A, K370E, Y407V, K409A |
| 45 | Q347K, S354C, E356K, E357K, T366W, D399W | Y349C, K360Q, T366S, L368A, K370E, Y407V, K409A, K439E |
| 46 | S354C, E356R, T366W, D399H | Y349C, T366S, L368A, Y407V, K409V, K439D |
| 47 | S354C, E356R, E357R, T366W | Y349C, T366S, L368A, K370N, Y407V, K439D |
| 48 | S354C, E357R, T366W, D399H | Y349C, T366S, L368A, K370N, Y407V, K409V |
| 49 | S354C, E356R, E357R, T366W, D399H | Y349C, T366S, L368A, K370N, Y407V, K409V, K439D |
| 50 | Q347K, S354C, T366W | Y349C, K360I, T366S, L368A, Y407V |
| 51 | Q347K, S354C, T366W | Y349C, K360L, T366S, L368A, Y407V |
| 52 | Q347K, S354C, T366W | Y349C, K360V, T366S, L368A, Y407V |
| 53 | Q347K, S354C, T366W | Y349C, K360F, T366S, L368A, Y407V |
| 54 | Q347K, S354C, T366W | Y349C, K360W, T366S, L368A, Y407V |
| 55 | Q347K, S354C, T366W | Y349C, K360Y, T366S, L368A, Y407V |
| 56 | Q347K, S354C, T366W | Y349C, K360H, T366S, L368A, Y407V |
| 57 | Q347I, S354C, T366W | Y349C, K360Q, T366S, L368A, Y407V |
| 58 | Q347L, S354C, T366W | Y349C, K360Q, T366S, L368A, Y407V |
| 59 | Q347V, S354C, T366W | Y349C, K360Q, T366S, L368A, Y407V |
| 60 | Q347F, S354C, T366W | Y349C, K360Q, T366S, L368A, Y407V |
| 61 | Q347W, S354C, T366W | Y349C, K360Q, T366S, L368A, Y407V |
| 62 | Q347Y, S354C, T366W | Y349C, K360Q, T366S, L368A, Y407V |
| 63 | Q347H, S354C, T366W | Y349C, K360Q, T366S, L368A, Y407V |
| 64 | S354C, E357K, T366W | Y349C, T366S, L368A, K3701, Y407V |
| 65 | S354C, E357K, T366W | Y349C, T366S, L368A, K370L, Y407V |
| 66 | S354C, E357K, T366W | Y349C, T366S, L368A, K370V, Y407V |
| 67 | S354C, E357K, T366W | Y349C, T366S, L368A, K370F, Y407V |
| 68 | S354C, E357K, T366W | Y349C, T366S, L368A, K370W, Y407V |
| 69 | S354C, E357K, T366W | Y349C, T366S, L368A, K370Y, Y407V |
| 70 | S354C, E357K, T366W | Y349C, T366S, L368A, K370H, Y407V |
| 71 | S354C, E3571, T366W | Y349C, T366S, L368A, K370E, Y407V |
| 72 | S354C, E357L, T366W | Y349C, T366S, L368A, K370E, Y407V |
| 73 | S354C, E357V, T366W | Y349C, T366S, L368A, K370E, Y407V |
| 74 | S354C, E357F, T366W | Y349C, T366S, L368A, K370E, Y407V |
| 75 | S354C, E357W, T366W | Y349C, T366S, L368A, K370E, Y407V |
| 76 | S354C, E357Y, T366W | Y349C, T366S, L368A, K370E, Y407V |
| 77 | S354C, E357H, T366W | Y349C, T366S, L368A, K370E, Y407V |
| 78 | S354C, E356K, T366W | Y349C, T366S, L368A, Y407V, K439I |
| 79 | S354C, E356K, T366W | Y349C, T366S, L368A, Y407V, K439L |
| 80 | S354C, E356K, T366W | Y349C, T366S, L368A, Y407V, K439V |
| 81 | S354C, E356K, T366W | Y349C, T366S, L368A, Y407V, K439F |
| 82 | S354C, E356K, T366W | Y349C, T366S, L368A, Y407V, K439W |
| 83 | S354C, E356K, T366W | Y349C, T366S, L368A, Y407V, K439Y |
| 84 | S354C, E356K, T366W | Y349C, T366S, L368A, Y407V, K439H |
| 85 | S354C, E3561, T366W | Y349C, T366S, L368A, Y407V, K439E |
| 86 | S354C, E356L, T366W | Y349C, T366S, L368A, Y407V, K439E |
| 87 | S354C, E356V, T366W | Y349C, T366S, L368A, Y407V, K439E |
| 88 | S354C, E356F, T366W | Y349C, T366S, L368A, Y407V, K439E |
| 89 | S354C, E356W, T366W | Y349C, T366S, L368A, Y407V, K439E |
| 90 | S354C, E356Y, T366W | Y349C, T366S, L368A, Y407V, K439E |
| 91 | S354C, E356H, T366W | Y349C, T366S, L368A, Y407V, K439E |
| 92 | S354C, T366W, D399W | Y349C, T366S, L368A, Y407V, K409Q |
| 93 | S354C, T366W, D399W | Y349C, T366S, L368A, Y407V, K409N |
| 94 | S354C, T366W, D399W | Y349C, T366S, L368A, Y407V, K409E |
| 95 | S354C, T366W, D399W | Y349C, T366S, L368A, Y407V, K409D |
| 96 | S354C, T366W, D399W | Y349C, T366S, L368A, Y407V, K409I |
| 97 | S354C, T366W, D399W | Y349C, T366S, L368A, Y407V, K409L |
| 98 | S354C, T366W, D399I | Y349C, T366S, L368A, Y407V, K409A |
| 99 | S354C, T366W, D399L | Y349C, T366S, L368A, Y407V, K409A |
| 100 | S354C, T366W, D399V | Y349C, T366S, L368A, Y407V, K409A |
| 101 | S354C, K360Q, T366W | Q347K, Y349C, T366S, L368A, Y407V |
| 102 | S354C, K360N, T366W | Q347K, Y349C, T366S, L368A, Y407V |
| 103 | S354C, K360E, T366W | Q347K, Y349C, T366S, L368A, Y407V |
| 104 | S354C, K360D, T366W | Q347K, Y349C, T366S, L368A, Y407V |
| 105 | S354C, K360Q, T366W | Q347R, Y349C, T366S, L368A, Y407V |
| 106 | S354C, K360N, T366W | Q347R, Y349C, T366S, L368A, Y407V |
| 107 | S354C, K360E, T366W | Q347R, Y349C, T366S, L368A, Y407V |
| 108 | S354C, K360D, T366W | Q347R, Y349C, T366S, L368A, Y407V |
| 109 | S354C, T366W, K370E | Y349C, E357K, T366S, L368A, Y407V |
| 110 | S354C, T366W, K370D | Y349C, E357K, T366S, L368A, Y407V |
| 111 | S354C, T366W, K370N | Y349C, E357K, T366S, L368A, Y407V |
| 112 | S354C, T366W, K370Q | Y349C, E357K, T366S, L368A, Y407V |
| 113 | S354C, T366W, K370E | Y349C, E357R, T366S, L368A, Y407V |
| 114 | S354C, T366W, K370D | Y349C, E357R, T366S, L368A, Y407V |
| 115 | S354C, T366W, K370N | Y349C, E357R, T366S, L368A, Y407V |
| 116 | S354C, T366W, K370Q | Y349C, E357R, T366S, L368A, Y407V |
| 117 | S354C, T366W, K439E | Y349C, E356K, T366S, L368A, Y407V |
| 118 | S354C, T366W, K439D | Y349C, E356K, T366S, L368A, Y407V |
| 119 | S354C, T366W, K439N | Y349C, E356K, T366S, L368A, Y407V |
| 120 | S354C, T366W, K439Q | Y349C, E356K, T366S, L368A, Y407V |
| 121 | S354C, T366W, K439E | Y349C, E356R, T366S, L368A, Y407V |
| 122 | S354C, T366W, K439D | Y349C, E356R, T366S, L368A, Y407V |
| 123 | S354C, T366W, K439N | Y349C, E356R, T366S, L368A, Y407V |
| 124 | S354C, T366W, K439Q | Y349C, E356R, T366S, L368A, Y407V |
| 125 | S354C, T366W, K409A | Y349C, T366S, L368A, D399W, Y407V |
| 126 | S354C, T366W, K409V | Y349C, T366S, L368A, D399F, Y407V |
| 127 | S354C, T366W, K409T | Y349C, T366S, L368A, D399F, Y407V |
| 128 | S354C, T366W, K409S | Y349C, T366S, L368A, D399F, Y407V |
| 129 | S354C, T366W, K409V | Y349C, T366S, L368A, D399Y, Y407V |
| 130 | S354C, T366W, K409T | Y349C, T366S, L368A, D399Y, Y407V |
| 131 | S354C, T366W, K409S | Y349C, T366S, L368A, D399Y, Y407V |
| 132 | S354C, T366W, K409V | Y349C, T366S, L368A, D399H, Y407V |
| 133 | S354C, T366W, K409T | Y349C, T366S, L368A, D399H, Y407V |
| 134 | S354C, T366W, K409S | Y349C, T366S, L368A, D399H, Y407V |
| 135 | S354C, K360Q, T366W, K370E | Q347K, Y349C, E357K, T366S, L368A, Y407V |
| 136 | S354C, K360Q, T366W, K439E | Q347K, Y349C, E356K, T366S, L368A, Y407V |
| 137 | S354C, T366W, K370E, K439E | Y349C, E356K, E357K, T366S, L368A, Y407V |
| 138 | S354C, K360Q, T366W, K409A | Q347K, Y349C, T366S, L368A, D399W, Y407V |
| 139 | S354C, T366W, K370E, K409A | Y349C, E357K, T366S, L368A, D399W, Y407V |
| 140 | S354C, T366W, K409A, K439E | Y349C, E356K, T366S, L368A, D399W, Y407V |
| 141 | S354C, K360Q, T366W, K370E, K439E | Q347K, Y349C, E356K, E357K, T366S, L368A, Y407V |
| 142 | S354C, T366W, K370E, K409A, K439E | Y349C, E356K, E357K, T366S, L368A, D399W, Y407V |
| 143 | S354C, K360Q, T366W, K409A, K439E | Q347K, Y349C, E356K, T366S, L368A, D399W, Y407V |
| 144 | S354C, K360Q, T366W, K370E, K409A | Q347K, Y349C, E357K, T366S, L368A, D399W, Y407V |
| 145 | S354C, K360Q, T366W, K370E, K409A, K439E | Q347K, Y349C, E356K, E357K, T366S, L368A, D399W, Y407V |
| 146 | S354C, T366W, K409V, K439D | Y349C, E356R, T366S, L368A, D399H, Y407V |
| 147 | S354C, T366W, K370N, K439D | Y349C, E356R, E357R, T366S, L368A, Y407V |
| 148 | S354C, T366W, K370N, K409V | Y349C, E357R, T366S, L368A, D399H, Y407V |
| 149 | S354C, T366W, K370N, K409V, K439D | Y349C, E356R, E357R, T366S, L368A, D399H, Y407V |
| 150 | S354C, K360I, T366W | Q347K, Y349C, T366S, L368A, Y407V |
| 151 | S354C, K360L, T366W | Q347K, Y349C, T366S, L368A, Y407V |
| 152 | S354C, T K360V, 366W | Q347K, Y349C, T366S, L368A, Y407V |
| 153 | S354C, K360F, T366W | Q347K, Y349C, T366S, L368A, Y407V |
| 154 | S354C, K360W, T366W | Q347K, Y349C, T366S, L368A, Y407V |
| 155 | S354C, K360Y, T366W | Q347K, Y349C, T366S, L368A, Y407V |
| 156 | S354C, K360H, T366W | Q347K, Y349C, T366S, L368A, Y407V |
| 157 | S354C, K360Q, T366W | Q347I, Y349C, T366S, L368A, Y407V |
| 158 | S354C, K360Q, T366W | Q347L, Y349C, T366S, L368A, Y407V |
| 159 | S354C, K360Q, T366W | Q347V, Y349C, T366S, L368A, Y407V |
| 160 | S354C, K360Q, T366W | Q347F, Y349C, T366S, L368A, Y407V |
| 161 | S354C, K360Q, T366W | Q347W, Y349C, T366S, L368A, Y407V |
| 162 | S354C, K360Q, T366W | Q347Y, Y349C, T366S, L368A, Y407V |
| 163 | S354C, K360Q, T366W | Q347H, Y349C, T366S, L368A, Y407V |
| 164 | S354C, T366W, K370I | Y349C, E357K, T366S, L368A, Y407V |
| 165 | S354C, T366W, K370L | Y349C, E357K, T366S, L368A, Y407V |
| 166 | S354C, T366W, K370V | Y349C, E357K, T366S, L368A, Y407V |
| 167 | S354C, T366W, K370F | Y349C, E357K, T366S, L368A, Y407V |
| 168 | S354C, T366W, K370W | Y349C, E357K, T366S, L368A, Y407V |
| 169 | S354C, T366W, K370Y | Y349C, E357K, T366S, L368A, Y407V |
| 170 | S354C, T366W, K370H | Y349C, E357K, T366S, L368A, Y407V |
| 171 | S354C, T366W, K370E | Y349C, E357I, T366S, L368A, Y407V |
| 172 | S354C, T366W, K370E | Y349C, E357L, T366S, L368A, Y407V |
| 173 | S354C, T366W, K370E | Y349C, E357V, T366S, L368A, Y407V |
| 174 | S354C, T366W, K370E | Y349C, E357F, T366S, L368A, Y407V |
| 175 | S354C, T366W, K370E | Y349C, E357W, T366S, L368A, Y407V |
| 176 | S354C, T366W, K370E | Y349C, E357Y, T366S, L368A, Y407V |
| 177 | S354C, T366W, K370E | Y349C, E357H, T366S, L368A, Y407V |
| 178 | S354C, T366W, K439I | Y349C, E356K, T366S, L368A, Y407V |
| 179 | S354C, T366W, K439L | Y349C, E356K, T366S, L368A, Y407V |
| 180 | S354C, T366W, K439V | Y349C, E356K, T366S, L368A, Y407V |
| 181 | S354C, T366W, K439F | Y349C, E356K, T366S, L368A, Y407V |
| 182 | S354C, T366W, K439W | Y349C, E356K, T366S, L368A, Y407V |
| 183 | S354C, T366W, K439Y | Y349C, E356K, T366S, L368A, Y407V |
| 184 | S354C, T366W, K439H | Y349C, E356K, T366S, L368A, Y407V |
| 185 | S354C, T366W, K439E | Y349C, E356I, T366S, L368A, Y407V |
| 186 | S354C, T366W, K439E | Y349C, E356L, T366S, L368A, Y407V |
| 187 | S354C, T366W, K439E | Y349C, E356V, T366S, L368A, Y407V |
| 188 | S354C, T366W, K439E | Y349C, E356F, T366S, L368A, Y407V |
| 189 | S354C, T366W, K439E | Y349C, E356W, T366S, L368A, Y407V |
| 190 | S354C, T366W, K439E | Y349C, E356Y, T366S, L368A, Y407V |
| 191 | S354C, T366W, K439E | Y349C, E356H, T366S, L368A, Y407V |
| 192 | S354C, T366W, K409Q | Y349C, T366S, L368A, D399W, Y407V |
| 193 | S354C, T366W, K409N | Y349C, T366S, L368A, D399W, Y407V |
| 194 | S354C, T366W, K409E | Y349C, T366S, L368A, D399W, Y407V |
| 195 | S354C, T366W, K409D | Y349C, T366S, L368A, D399W, Y407V |
| 196 | S354C, T366W, K409I | Y349C, T366S, L368A, D399W, Y407V |
| 197 | S354C, T366W, K409L | Y349C, T366S, L368A, D399W, Y407V |
| 198 | S354C, T366W, K409A | Y349C, T366S, L368A, D3991, Y407V |
| 199 | S354C, T366W, K409A | Y349C, T366S, L368A, D399L, Y407V |

Still another aspect of the present invention relates to a fusion protein including the heterodimer.

The heterodimer may be bound to one terminus of a polypeptide or protein, for example, a peptide, a cytokine such as IL-2, IL-10, IL-12, GCSF, or GM-CSF, a chemokine such as RANTES, CXCL9, CXCL10, or CXCL12, a hormone, an immune checkpoint protein such as CTLA-4, TNFR1, TNFRII, TNFSF, or TNFRSF, or a blood factor.

The heterodimer may be bound to the N-terminus or C-terminus of a polypeptide or protein. In some cases, the heterodimer may be linked to a polypeptide or protein by a linker. The linker may be a peptide linker and may include about 5-25 aa residues, particularly about 5-10 aa residues. For example, hydrophilic amino acids such as, but not limited to, glycine and/or serine may be included.

In the bispecific antibody format according to the present invention, the heavy chain and light chain of the first arm that binds to the first antigen are configured as follows. The heavy chain of the first arm is composed in the order of VH-CH3a-Linker-CH1a-Hinge-CH2-CH3b. As the CH1a domain, an IgG1 CH1 domain may be used. The CH3a domain may include mutations in the hole and mutations to form a disulfide bond with the CH3c of the light chain. The CH3b domain may include mutations for disulfide bond formation. An elbow sequence AS may be added between the VH region and the CH3a region, as necessary.

Taking into consideration mutations having biologically equivalent activity, the substitutions according to the present invention are to be construed as including substitutions with the sequences described herein as well as with sequences exhibiting substantial identity thereto.

Substantial identity means a sequence that exhibits at least 61% homology, more preferably 70% or more homology, even more preferably 80% or more homology, most preferably 90%, 91%, 92%, 93%, 94%, 95%, 96%, 97%, 98%, 99% or more homology when the sequence of the present invention and any other sequence are aligned so as to correspond to each other as closely as possible and the aligned sequences are analyzed using an algorithm commonly used in the art. Alignment methods for sequence comparison are known in the art. The NCBI Basic Local Alignment Search Tool (BLAST) may be accessed from NBCI or the like, and is available in conjunction with sequencing programs such as blastp, blasm, blastx, tblastn, and tblastx on the Internet. BLAST may be accessed at www.ncbi.nlm.nih.gov/BLAST/. A method for comparing sequence homology using this program may be found at www.ncbi.nlm.nih.gov/BLAST/blast_help.html.

A better understanding of the present invention may be obtained through the following examples. These examples are merely set forth to illustrate the present invention and are not to be construed as limiting the scope of the present invention, as will be apparent to those skilled in the art. Accordingly, the substantial scope of the present invention will be defined by the appended claims and equivalents thereto.

### Example 1. Design and engineering of S-DUAL 2G antibody

### 1.1. Introduction of CH3 domain amino acid residue variants

A bispecific antibody format that simultaneously binds to a first antigen and a second antigen is configured as follows. The bispecific antibody format is formed by the assembly of a total of four polypeptides, namely two heavy chains (H) and two light chains (L). The heavy and light chains of the first arm that binds to the first antigen are configured as follows. The heavy chain is composed of VH-CH3D-CH1a-Hinge-CH2-CH3B. Here, CH3B and CH3D may include sequences of the IgG1 CH3 domain, and the CH1a region may include sequences of the IgG1 CH1 domain. Among the domains that make up the heavy chain, the IgG1 CH3B and CH3D domains include mutations in knob (T366W) or hole (T366S/L368A/Y407V). The IgG1 CH3B and CH3D domains may also include mutations that introduce cysteine residues to form inter-domain disulfide bonds, namely knob (S354C/T366W) or knob (Y349C/T366W) or hole (S354C/T366S/L368A/Y407V) or hole (Y349C/T366S/L368A/Y407V). The IgG1 CH3 mutation candidates were identified using the "protein interaction analysis" panel of Bioluminate^{®} (Schrodinger, LLC), by screening amino acid residues that play a major role in the binding between the CH3A-CH3B domains that constitute the heavy chain constant region, and applying replaceable amino acid mutations designed to avoid steric clashes with the side chain structures of neighboring amino acid residues. Based on the above candidates, four amino acid residue mutation pairs were selected through structural modeling, which had high interaction energy values between heterodimers (CH3A-CH3B) but low interaction energy values between homodimers (CH3A-CH3A, CH3B-CH3B). Afterwards, amino acid residue mutations were introduced into the bispecific antibody by incorporating single, double, or triple combinations of mutations into the CH3 domain pair of the heavy chain constant region.

The light chain of the first arm that binds to the first antigen is composed of VL-CH3C-CLb. Here, CH3C represents the IgG1 CH3 domain, which contains mutations in knob (T366W) or hole (T366S/L368A/Y407V). The IgG1 CH3 domain may also include mutations that introduce cysteine residues for disulfide bonding, namely knob (S354C/T366W) or knob (Y349C/T366W) or hole (S354C/T366S/L368A/Y407V) or hole (Y349C/T366S/L368A/Y407V). The heavy and light chains of the second arm that binds to the second antigen are configured as follows. The heavy chain is composed of VH-CH1-Hinge-CH2-CH3A. The IgG1 CH3A domain includes mutations in knob (T366W) or hole (T366S/L368A/Y407V). The IgG1 CH3A domain may also include mutations that introduce cysteine residues for disulfide bonding, namely knob (S354C/T366W) or knob (Y349C/T366W) or hole (S354C/T366S/L368A/Y407V) or hole (Y349C/T366S/L368A/Y407V). The light chain of the second arm is composed of VL-CLb. Based on the bispecific antibody format described above, additional IgG1 CH3 mutations were intended to be introduced to increase the yield of heterodimer formation. The IgG1 CH3 mutation candidates, including four amino acid residue mutations in single combinations, were introduced into the CH3B and CH3D domains in the heavy chain VH-CH3D-CH1a-Hinge-CH2-CH3B of the first arm that binds to the first antigen, and into the CH3C domain in the light chain VL-CH3C-CLb.

**[Table 1]**

| CH3 mutation candidates obtained through *in-silico* simulation | | | | | | | | | | | |
|---|---|---|---|---|---|---|---|---|---|---|---|
| **CH3A** | | | **CH3B** | | | **CH3C** | | | **CH3D** | | |
| Q | 347 | K | K | 360 | Q | Q | 347 | K | K | 360 | Q |
| Q | 347 | K | K | 360 | N | Q | 347 | K | K | 360 | N |
| Q | 347 | K | K | 360 | E | Q | 347 | K | K | 360 | E |
| Q | 347 | K | K | 360 | D | Q | 347 | K | K | 360 | D |
| Q | 347 | R | K | 360 | Q | Q | 347 | R | K | 360 | Q |
| Q | 347 | R | K | 360 | N | Q | 347 | R | K | 360 | N |
| Q | 347 | R | K | 360 | E | Q | 347 | R | K | 360 | E |
| Q | 347 | R | K | 360 | D | Q | 347 | R | K | 360 | D |
| E | 357 | K | K | 370 | E | E | 357 | K | K | 370 | E |
| E | 357 | K | K | 370 | D | E | 357 | K | K | 370 | D |
| E | 357 | K | K | 370 | N | E | 357 | K | K | 370 | N |
| E | 357 | K | K | 370 | Q | E | 357 | K | K | 370 | Q |
| E | 357 | R | K | 370 | E | E | 357 | R | K | 370 | E |
| E | 357 | R | K | 370 | D | E | 357 | R | K | 370 | D |
| E | 357 | R | K | 370 | N | E | 357 | R | K | 370 | N |
| E | 357 | R | K | 370 | Q | E | 357 | R | K | 370 | Q |
| E | 356 | K | K | 439 | E | E | 356 | K | K | 439 | E |
| E | 356 | K | K | 439 | D | E | 356 | K | K | 439 | D |
| E | 356 | K | K | 439 | N | E | 356 | K | K | 439 | N |
| E | 356 | K | K | 439 | Q | E | 356 | K | K | 439 | Q |
| E | 356 | R | K | 439 | E | E | 356 | R | K | 439 | E |
| E | 356 | R | K | 439 | D | E | 356 | R | K | 439 | D |
| E | 356 | R | K | 439 | N | E | 356 | R | K | 439 | N |
| E | 356 | R | K | 439 | Q | E | 356 | R | K | 439 | Q |
| D | 399 | W | K | 409 | A | D | 399 | W | K | 409 | A |
| D | 399 | F | K | 409 | V | D | 399 | F | K | 409 | V |
| D | 399 | F | K | 409 | T | D | 399 | F | K | 409 | T |
| D | 399 | F | K | 409 | S | D | 399 | F | K | 409 | S |
| D | 399 | Y | K | 409 | V | D | 399 | Y | K | 409 | V |
| D | 399 | Y | K | 409 | T | D | 399 | Y | K | 409 | T |
| D | 399 | Y | K | 409 | S | D | 399 | Y | K | 409 | S |
| D | 399 | H | K | 409 | V | D | 399 | H | K | 409 | V |
| D | 399 | H | K | 409 | T | D | 399 | H | K | 409 | T |
| D | 399 | H | K | 409 | S | D | 399 | H | K | 409 | S |

### 1.2 Design by anti-VEGF x anti-HER2 bispecific antibody

The first antigen was determined to be HER2 protein, and the heavy chain variable region amino acid sequence and the light chain variable region amino acid sequence of trastuzumab binding to HER2 were used for the VH or VL of the bispecific antibody format. The second antigen was determined to be a VEGF-A target, and the heavy chain variable region amino acid sequence and the light chain variable region amino acid sequence of bevacizumab were used for the VH or VL of the bispecific antibody format. Amino acid sequence information was obtained through https:/go.drugbank.com/.

**[Table 2]**

| Information on bispecific antibodies with amino acid residue mutations in Fc and Fab CH3 domain pairs | | | | |
|---|---|---|---|---|
| **Name** | **CH3A** | **CH3B** | **CH3C** | **CH3D** |
| Q-SBL-100 | Y349C, T366W | S354C, T366S, L368A, Y407V | Y349C, T366W | S354C, T366S, L368A, Y407V |
| Q-SBL-101 | Y349C, E356R, T366W | S354C, T366S, L368A, Y407V, K439D | Y349C, T366W | S354C, T366S, L368A, Y407V |
| Q-SBL-102 | Y349C, T366W, D399H | S354C, T366S, L368A, Y407V, K409V | Y349C, T366W | S354C, T366S, L368A, Y407V |
| Q-SBL-103 | Y349C, E357R, T366W | S354C, T366S, L368A, Y407V, K370N | Y349C, T366W | S354C, T366S, L368A, Y407V |
| Q-SBL-104 | Y349C, E356R, T366W, D399H | S354C, T366S, L368A, Y407V, K409V, K439D | Y349C, T366W | S354C, T366S, L368A, Y407V |
| Q-SBL-105 | Y349C, E356R, E357R, T366W | S354C, T366S, L368A, K370N, Y407V, K439D | Y349C, T366W | S354C, T366S, L368A, Y407V |
| Q-SBL-106 | Y349C, E357R, T366W, D399H | S354C, T366S, L368A, K370N, Y407V, K409V | Y349C, T366W | S354C, T366S, L368A, Y407V |
| Q-SBL-107 | Y349C, E356R, E357R, T366W, D399H | S354C, T366S, L368A, K370N, Y407V, K409V, K439D | Y349C, T366W | S354C, T366S, L368A, Y407V |
| Q-SBL-108 | Y349C, T366W | S354C, T366S, L368A, Y407V | S354C, T366W | Y349C, T366S, L368A, Y407V |
| Q-SBL-109 | Y349C, E356R, T366W | S354C, T366S, L368A, Y407V, K439D | S354C, T366W | Y349C, T366S, L368A, Y407V |
| Q-SBL-110 | S354C, E356R, T366W | Y349C, T366S, L368A, Y407V, K439D | Y349C, T366W, D399H | S354C, T366S, L368A, Y407V, K409V |
| Q-SBL-111 | Y349C, E356R, T366W | S354C, T366S, L368A, Y407V, K439D | S354C, E357R, T366W | Y349C, T366S, L368A, Y407V, K370N |
| Q-SBL-112 | S354C, E356R, T366W | Y349C, T366S, L368A, Y407V, K439D | S354C, T366W, K409V | Y349C, T366S, L368A, Y407V, D399H |
| Q-SBL-113 | S354C, E356R, T366W | Y349C, T366S, L368A, Y407V, K439D | S354C, T366W, K370N | Y349C, E357R, T366S, L368A, Y407V |
| Q-SBL-114 | Y349C, T366W, D399H | S354C, T366S, L368A, Y407V, K409V | S354C, T366W | Y349C, T366S, L368A, Y407V |
| Q-SBL-115 | Y349C, E357R, T366W | S354C, T366S, L368A, K370N, Y407V | S354C, T366W | Y349C, T366S, L368A, Y407V |
| Q-SBL-116 | Y349C, E356K, T366W | S354C, T366S, L368A, Y407V, K439N | S354C, T366W | Y349C, T366S, L368A, Y407V |
| Q-SBL-117 | Y349C, E356K, T366W | S354C, T366S, L368A, Y407V, K439Q | S354C, T366W | Y349C, T366S, L368A, Y407V |
| Q-SBL-118 | Y349C, E356K, T366W | S354C, T366S, L368A, Y407V, K439N | S354C, E357R, T366W | Y349C, T366S, L368A, Y407V, K370N |
| Q-SBL-119 | Y349C, E356K, T366W | S354C, T366S, L368A, Y407V, K439Q | S354C, E357R, T366W | Y349C, T366S, L368A, Y407V, K370N |
| Q-SBL-120 | Y349C, T366W, D399H | S354C, T366S, L368A, Y407V, K409V | S354C, E356R, T366W | Y349C, T366S, L368A, Y407V, K439D |
| Q-SBL-121 | Y349C, T366W, D399H | S354C, T366S, L368A, Y407V, K409V | S354C, T366W, D399H | Y349C, T366S, L368A, Y407V, K409V |
| Q-SBL-122 | Y349C, E356R, T366W | S354C, T366S, L368A, Y407V, K439N | S354C, T366W | Y349C, T366S, L368A, Y407V |
| Q-SBL-123 | Y349C, E356R, T366W | S354C, T366S, L368A, Y407V, K439Q | S354C, T366W | Y349C, T366S, L368A, Y407V |
| Q-SBL-124 | Y349C, E356R, T366W | S354C, T366S, L368A, Y407V, K439N | S354C, E357R, T366W | Y349C, T366S, L368A, Y407V, K370N |
| Q-SBL-125 | Y349C, E356R, T366W | S354C, T366S, L368A, Y407V, K439Q | S354C, E357R, T366W | Y349C, T366S, L368A, Y407V, K370N |
| Q-SBL-126 | Y349C, T366W, D399H | S354C, T366S, L368A, Y407V, K409V | S354C, E357R, T366W | Y349C, T366S, L368A, Y407V, K370N |
| Q-SBL-127 | Y349C, T366W, D399F | S354C, T366S, L368A, Y407V, K409V | S354C, T366W | Y349C, T366S, L368A, Y407V |
| Q-SBL-128 | Y349C, T366W, D399F | S354C, T366S, L368A, Y407V, K409T | S354C, T366W | Y349C, T366S, L368A, Y407V |
| Q-SBL-129 | Y349C, T366W, D399F | S354C, T366S, L368A, Y407V, K409V | S354C, E357R, T366W | Y349C, T366S, L368A, Y407V, K370N |
| Q-SBL-130 | Y349C, T366W, D399F | S354C, T366S, L368A, Y407V, K409V | S354C, E356R, T366W | Y349C, T366S, L368A, Y407V, K439D |
| Q-SBL-131 | Y349C, T366W, D399F | S354C, T366S, L368A, Y407V, K409V | S354C, T366W, D399H | Y349C, T366S, L368A, Y407V, K409V |
| Q-SBL-132 | Y349C, T366W, K409V | S354C, T366S, L368A, D399F, Y407V | S354C, T366W | Y349C, T366S, L368A, Y407V |
| Q-SBL-133 | Y349C, T366W, K409T | S354C, T366S, L368A, D399F, Y407V | S354C, T366W | Y349C, T366S, L368A, Y407V |
| Q-SBL-134 | Y349C, T366W, K409S | S354C, T366S, L368A, D399F, Y407V | S354C, T366W | Y349C, T366S, L368A, Y407V |
| Q-SBL-135 | Y349C, T366W, K409V | S354C, T366S, L368A, D399Y, Y407V | S354C, T366W | Y349C, T366S, L368A, Y407V |
| Q-SBL-136 | Y349C, T366W, K409T | S354C, T366S, L368A, D399Y, Y407V | S354C, T366W | Y349C, T366S, L368A, Y407V |
| Q-SBL-137 | Y349C, T366W, K409S | S354C, T366S, L368A, D399Y, Y407V | S354C, T366W | Y349C, T366S, L368A, Y407V |
| Q-SBL-138 | Y349C, T366W, K409V | S354C, T366S, L368A, D399H, Y407V | S354C, T366W | Y349C, T366S, L368A, Y407V |
| Q-SBL-139 | Y349C, T366W, K409T | S354C, T366S, L368A, D399H, Y407V | S354C, T366W | Y349C, T366S, L368A, Y407V |
| Q-SBL-140 | Y349C, T366W, K409S | S354C, T366S, L368A, D399H, Y407V | S354C, T366W | Y349C, T366S, L368A, Y407V |
| Q-SBL-141 | Y349C, T366W, K409A | S354C, T366S, L368A, D399W, Y407V | S354C, T366W | Y349C, T366S, L368A, Y407V |
| Q-SBL-142 | S354C, T366W, K409V | S354C, T366S, L368A, D399F, Y407V | Y349C, T366W, D399H | S354C, T366S, L368A, Y407V, K409V |

The specific composition of each candidate is shown in Table 3 below.

**[Table 3]**

| Heavy and light chain sequences of each candidate | | |
|---|---|---|
| Composition | Sequence | No. |
| Q-SBL-100 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCTLPPSREEMTKNQVSLSCAVKGFYPSDI AVEWESNGQPENNYKTTPPVLDSDGSFFLVSKLTVDKSRWQQGNV FSCSVMHEALHNHYTQKSLSLSPGK | 3 |
| Q-SBL-100 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 4 |
| Q-SBL-101 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQDSLSLSPGK | 5 |
| Q-SBL-101 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 6 |
| Q-SBL-102 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQKSLSLSPGK | 7 |
| Q-SBL-102 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLHSD GSFFLYSKLTVDKSRWQQGNVFSCSVMHEALHNHYTQKSLSLSPG K | 8 |
| Q-SBL-103 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVNGFYPSDIAVEW ESNGQPENNYKTTPPVLDSDGSFFLVSKLTVDKSRWQQGNVFSCS VMHEALHNHYTQKSLSLSPGK | 9 |
| Q-SBL-103 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE RMTKNQVSLWCLVKGFYPSDIAVEWESNGQPENNYKTTPPVLDSD GSFFLYSKLTVDKSRWQQGNVFSCSVMHEALHNHYTQKSLSLSPG K | 10 |
| Q-SBL-104 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP | 11 |
| | KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQDSLSLSPGK | |
| Q-SBL-104 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLHSD GSFFLYSKLTVDKSRWQQGNVFSCSVMHEALHNHYTQKSLSLSPG K | 12 |
| Q-SBL-105 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVNGFYPSDIAVEW ESNGQPENNYKTTPPVLDSDGSFFLVSKLTVDKSRWQQGNVFSCS VMHEALHNHYTQDSLSLSPGK | 13 |
| Q-SBL-105 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR RMTKNQVSLWCLVKGFYPSDIAVEWESNGQPENNYKTTPPVLDSD GSFFLYSKLTVDKSRWQQGNVFSCSVMHEALHNHYTQKSLSLSPG K | 14 |
| Q-SBL-106 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVNGFYPSDIAVEW ESNGQPENNYKTTPPVLDSDGSFFLVSVLTVDKSRWQQGNVFSCS VMHEALHNHYTQKSLSLSPGK | 15 |
| Q-SBL-106 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP | 16 |
| | LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE RMTKNQVSLWCLVKGFYPSDIAVEWESNGQPENNYKTTPPVLHSD GSFFLYSKLTVDKSRWQQGNVFSCSVMHEALHNHYTQKSLSLSPG K | |
| Q-SBL-107 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVNGFYPSDIAVEW ESNGQPENNYKTTPPVLDSDGSFFLVSVLTVDKSRWQQGNVFSCS VMHEALHNHYTQDSLSLSPGK | 17 |
| Q-SBL-107 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR RMTKNQVSLWCLVKGFYPSDIAVEWESNGQPENNYKTTPPVLHSD GSFFLYSKLTVDKSRWQQGNVFSCSVMHEALHNHYTQKSLSLSPG K | 18 |
| Q-SBL-108 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQKSLSLSPGK | 19 |
| Q-SBL-108 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 20 |
| Q-SBL-109 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQDSLSLSPGK | 21 |
| Q-SBL-109 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 22 |
| Q-SBL-110 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSVLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQDSLSLSPGK | 23 |
| Q-SBL-110 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 24 |
| Q-SBL-111 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVNGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP | 25 |
| | KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQDSLSLSPGK | |
| Q-SBL-111 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 26 |
| Q-SBL-112 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLH SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQDSLSLSPGK | 27 |
| Q-SBL-112 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 28 |
| Q-SBL-113 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC RERMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQDSLSLSPGK | 29 |
| Q-SBL-113 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP | 30 |
| | LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | |
| Q-SBL-114 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQKSLSLSPGK | 31 |
| Q-SBL-114 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLHSD GSFFLYSKLTVDKSRWQQGNVFSCSVMHEALHNHYTQKSLSLSPG K | 32 |
| Q-SBL-115 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVNGFYPSDIAVEW ESNGQPENNYKTTPPVLDSDGSFFLVSKLTVDKSRWQQGNVFSCS VMHEALHNHYTQKSLSLSPGK | 33 |
| Q-SBL-115 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE RMTKNQVSLWCLVKGFYPSDIAVEWESNGQPENNYKTTPPVLDSD GSFFLYSKLTVDKSRWQQGNVFSCSVMHEALHNHYTQKSLSLSPG K | 34 |
| Q-SBL-116 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQNSLSLSPGK | 35 |
| Q-SBL-116 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRK EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 36 |
| Q-SBL-117 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQQSLSLSPGK | 37 |
| Q-SBL-117 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRK EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 38 |
| Q-SBL-118 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVNGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP | 39 |
| | KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQNSLSLSPGK | |
| Q-SBL-118 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRK EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 40 |
| Q-SBL-119 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVNGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQQSLSLSPGK | 41 |
| Q-SBL-119 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRK EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 42 |
| Q-SBL-120 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSS ASPREPQVCTLPPSREEMTKNQVSLSCAVKGFYPSDIAVEWESNGQ PENNYKTTPPVLDSDGSFFLVSKLTVDKSRWQQGNVFSCSVMHEA LHNHYTQDSLSLSPGKGGGGS ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKV EPKSSDKTHTCPPCP APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAK GQPREPQVYTLPPCREEMTKNQVSLSCAVKGFYPSDIAVEWESNG QPENNYKTTPPVLDSDGSFFLVSVLTVDKSRWQQGNVFSCSVMHE ALHNHYTQKSLSLSPGK | 43 |
| Q-SBL-120 HC (Second arm heavy chain region) | EVQLVESGGGLVQPGGSLRLSCAASGYTFTNYGMNWVRQAPGKG | 44 |
| | LEWVGWINTYTGEPTYAADFKRRFTFSLDTSKSTAYLQMNSLRAE DTAVYYCAKYPHYYGSSHWYFDVWGQGTLVTVSS ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKV EPKSCDKTHTCPPCP APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAK GQPREPQVCTLPPSREEMTKNQVSLWCLVKGFYPSDIAVEWESNG QPENNYKTTPPVLHSDGSFFLYSKLTVDKSRWQQGNVFSCSVMHE ALHNHYTQKSLSLSPGK | |
| Q-SBL-121 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSS ASPREPQVCTLPPSREEMTKNQVSLSCAVKGFYPSDIAVEWESNGQ PENNYKTTPPVLDSDGSFFLVSVLTVDKSRWQQGNVFSCSVMHEA LHNHYTQKSLSLSPGK GGGGS ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKV EPKSSDKTHTCPPCP APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAK GQPREPQVYTLPPCREEMTKNQVSLSCAVKGFYPSDIAVEWESNG QPENNYKTTPPVLDSDGSFFLVSVLTVDKSRWQQGNVFSCSVMHE ALHNHYTQKSLSLSPGK | 45 |
| Q-SBL-121 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSS ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKV EPKSCDKTHTCPPCP APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAK GQPREPQVCTLPPSREEMTKNQVSLWCLVKGFYPSDIAVEWESNG QPENNYKTTPPVLHSDGSFFLYSKLTVDKSRWQQGNVFSCSVMHE ALHNHYTQKSLSLSPGK | 46 |
| Q-SBL-122 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQNSLSLSPGK | 47 |
| Q-SBL-122 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS | 48 |
| | CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | |
| Q-SBL-123 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQQSLSLSPGK | 49 |
| Q-SBL-123 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 50 |
| Q-SBL-124 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVNGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQNSLSLSPGK | 51 |
| Q-SBL-124 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 52 |
| Q-SBL-125 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED | 53 |
| | TAVYYCSRWGGDGFYAMDYWGQGTLVTVSSASPREPQVCTLPPS REEMTKNQVSLSCAVNGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSKLTVDKSRWQQGNVFSCS VMHEALHNHYTQQSLSLSPGK | |
| Q-SBL-125 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRR EMTKNQVSLWCLVKGFYPSDIAVEWESNGQPENNYKTTPPVLDSD GSFFLYSKLTVDKSRWQQGNVFSCSVMHEALHNHYTQKSLSLSPG K | 54 |
| Q-SBL-126 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVNGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQKSLSLSPGK | 55 |
| Q-SBL-126 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLHSD GSFFLYSKLTVDKSRWQQGNVFSCSVMHEALHNHYTQKSLSLSPG K | 56 |
| Q-SBL-127 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI | 57 |
| | SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQKSLSLSPGK | |
| Q-SBL-127 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLFSD GSFFLYSKLTVDKSRWQQGNVFSCSVMHEALHNHYTQKSLSLSPG K | 58 |
| Q-SBL-128 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSTLTVDKSRWQQGNVFSCSV MHEALHNHYTQKSLSLSPGK | 59 |
| Q-SBL-128 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLFSD GSFFLYSKLTVDKSRWQQGNVFSCSVMHEALHNHYTQKSLSLSPG K | 60 |
| Q-SBL-129 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQDSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQKSLSLSPGK | 61 |
| Q-SBL-129 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS | 62 |
| | CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLFSD GSFFLYSKLTVDKSRWQQGNVFSCSVMHEALHNHYTQKSLSLSPG K | |
| Q-SBL-130 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQDSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQKSLSLSPGK | 63 |
| Q-SBL-130 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLFSD GSFFLYSKLTVDKSRWQQGNVFSCSVMHEALHNHYTQKSLSLSPG K | 64 |
| Q-SBL-131 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSVLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLDSDGSFFLVSVLTVDKSRWQQGNVFSCS VMHEALHNHYTQKSLSLSPGK | 65 |
| Q-SBL-131 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLFSD GSFFLYSKLTVDKSRWQQGNVFSCSVMHEALHNHYTQKSLSLSPG K | 66 |
| Q-SBL-132 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED | 67 |
| | TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLFSDGSFFLVSKLTVDKSRWQQGNVFSCSV MHEALHNHYTQKSLSLSPGK | |
| Q-SBL-132 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSVLTVDKSRWQQGNVFSCSVMHEALHNHYTQKSLSLSPG K | 68 |
| Q-SBL-133 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLFSDGSFFLVSKLTVDKSRWQQGNVFSCSV MHEALHNHYTQKSLSLSPGK | 69 |
| Q-SBL-133 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSTLTVDKSRWQQGNVFSCSVMHEALHNHYTQKSLSLSPG K | 70 |
| Q-SBL-134 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI | 71 |
| | SKAKGQPREPQVYTLPPCREEMTKNQVSLSCAVKGFYPSDIAVEW ESNGQPENNYKTTPPVLFSDGSFFLVSKLTVDKSRWQQGNVFSCSV MHEALHNHYTQKSLSLSPGK | |
| Q-SBL-134 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSSLTVDKSRWQQGNVFSCSVMHEALHNHYTQKSLSLSPG K | 72 |
| Q-SBL-135 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLYSDGSFFLVSKLTVDKSRWQQGNVFSCS VMHEALHNHYTQKSLSLSPGK | 73 |
| Q-SBL-135 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSVLTVDKSRWQQGNVFSCSVMHEALHNHYTQKSLSLSPG K | 74 |
| Q-SBL-136 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLYSDGSFFLVSKLTVDKSRWQQGNVFSCS VMHEALHNHYTQKSLSLSPGK | 75 |
| Q-SBL-136 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS | 76 |
| | CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSTLTVDKSRWQQGNVFSCSVMHEALHNHYTQKSLSLSPG K | |
| Q-SBL-137 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLYSDGSFFLVSKLTVDKSRWQQGNVFSCS VMHEALHNHYTQKSLSLSPGK | 77 |
| Q-SBL-137 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSSLTVDKSRWQQGNVFSCSVMHEALHNHYTQKSLSLSPG K | 78 |
| Q-SBL-138 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLHSDGSFFLVSKLTVDKSRWQQGNVFSCS VMHEALHNHYTQKSLSLSPGK | 79 |
| Q-SBL-138 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSVLTVDKSRWQQGNVFSCSVMHEALHNHYTQKSLSLSPG K | 80 |
| Q-SBL-139 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED | 81 |
| | TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLHSDGSFFLVSKLTVDKSRWQQGNVFSCS VMHEALHNHYTQKSLSLSPGK | |
| Q-SBL-139 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSTLTVDKSRWQQGNVFSCSVMHEALHNHYTQKSLSLSPG K | 82 |
| Q-SBL-140 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLSCAVKGFYPSDIAVEW ESNGQPENNYKTTPPVLHSDGSFFLVSKLTVDKSRWQQGNVFSCS VMHEALHNHYTQKSLSLSPGK | 83 |
| Q-SBL-140 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSSLTVDKSRWQQGNVFSCSVMHEALHNHYTQKSLSLSPG K | 84 |
| Q-SBL-141 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVCTLPPS REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI | 85 |
| | SKAKGQPREPQVYTLPPCREEMTKNQVSLSCAVKGFYPSDIAVEW ESNGQPENNYKTTPPVLWSDGSFFLVSKLTVDKSRWQQGNVFSCS VMHEALHNHYTQKSLSLSPGK | |
| Q-SBL-141 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRE EMTKNQVSLWCLVKGFYPSDIAVEWESNGQPENNYKTTPPVLDSD GSFFLYSALTVDKSRWQQGNVFSCSVMHEALHNHYTQKSLSLSPG K | 86 |
| Q-SBL-142 HC (First arm heavy chain region) | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASPREPQVYTLPPC REEMTKNQVSLSCAVKGFYPSDIAVEWESNGQPENNYKTTPPVLD SDGSFFLVSKLTVDKSRWQQGNVFSCSVMHEALHNHYTQKSLSLS PGKGGGGSASTKGPSVFPLAPSSKSTSGGTAALGCLVKDYFPEPVT VSWNSGALTSGVHTFPAVLQSSGLYSLSSVVTVPSSSLGTQTYICN VNHKPSNTKVDKKVEPKSSDKTHTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVCTLPPSREEMTKNQVSLSCAVKGFYPSDIAVEWE SNGQPENNYKTTPPVLFSDGSFFLVSKLTVDKSRWQQGNVFSCSV MHEALHNHYTQKSLSLSPGK | 87 |
| Q-SBL-142 HC (Second arm heavy chain region) | EVQLVESGGGLVQPGGSLRLSCAASGYTFTNYGMNWVRQAPGKG LEWVGWINTYTGEPTYAADFKRRFTFSLDTSKSTAYLQMNSLRAE DTAVYYCAKYPHYYGSSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPCRE EMTKNQVSLWCLVKGFYPSDIAVEWESNGQPENNYKTTPPVLDSD GSFFLYSVLTVDKSRWQQGNVFSCSVMHEALHNHYTQKSLSLSPG K | 88 |
| Q-SBL-100 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 89 |
| Q-SBL-100 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 90 |
| Q-SBL-101 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP | 91 |
| | PSDEQLKSGTASVVCLLNNFYPREAKVQWKVDNALQSGNSQESVT EQDSKDSTYSLSSTLTLSKADYEKHKVYACEVTHQGLSSPVTKSFN RGES | |
| Q-SBL-101 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 92 |
| Q-SBL-102 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 93 |
| Q-SBL-102 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 94 |
| Q-SBL-103 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 95 |
| Q-SBL-103 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 96 |
| Q-SBL-104 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 97 |
| Q-SBL-104 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 98 |
| Q-SBL-105 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 99 |
| Q-SBL-105 LC (Second arm light chain region) | DIQMTQSPSSLSASVGDRVTITCSASQDISNYLNWYQQKPGKAPKV | 100 |
| | LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | |
| Q-SBL-106 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 101 |
| Q-SBL-106 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 102 |
| Q-SBL-107 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 103 |
| Q-SBL-107 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 104 |
| Q-SBL-108 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 105 |
| Q-SBL-108 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 106 |
| Q-SBL-109 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 107 |
| Q-SBL-109 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 108 |
| Q-SBL-110 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLHSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 109 |
| Q-SBL-110 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 110 |
| Q-SBL-111 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCRERMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 111 |
| Q-SBL-111 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 112 |
| Q-SBL-112 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSVLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 113 |
| Q-SBL-112 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 114 |
| Q-SBL-113 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVNGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 115 |
| Q-SBL-113 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 116 |
| Q-SBL-114 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ | 117 |
| | QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | |
| Q-SBL-114 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 118 |
| Q-SBL-115 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 119 |
| Q-SBL-115 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 120 |
| Q-SBL-116 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 121 |
| Q-SBL-116 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 122 |
| Q-SBL-117 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 123 |
| Q-SBL-117 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 124 |
| Q-SBL-118 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCRERMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 125 |
| Q-SBL-118 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 126 |
| Q-SBL-119 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCRERMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 127 |
| Q-SBL-119 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 128 |
| Q-SBL-120 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCRREMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 129 |
| Q-SBL-120 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 130 |
| Q-SBL-121 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLHSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 131 |
| Q-SBL-121 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 132 |
| Q-SBL-122 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 133 |
| Q-SBL-122 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY | 134 |
| | EKHKVYACEVTHQGLSSPVTKSFNRGEC | |
| Q-SBL-123 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 135 |
| Q-SBL-123 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 136 |
| Q-SBL-124 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCRERMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 137 |
| Q-SBL-124 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 138 |
| Q-SBL-125 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCRERMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 139 |
| Q-SBL-125 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 140 |
| Q-SBL-126 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 141 |
| Q-SBL-126 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 142 |
| Q-SBL-127 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF | 143 |
| | YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | |
| Q-SBL-127 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 144 |
| Q-SBL-128 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 145 |
| Q-SBL-128 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 146 |
| Q-SBL-129 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 147 |
| Q-SBL-129 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 148 |
| Q-SBL-130 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCRREMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 149 |
| Q-SBL-130 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 150 |
| Q-SBL-131 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLHSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN | 151 |
| | RGES | |
| Q-SBL-131 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 152 |
| Q-SBL-132 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 153 |
| Q-SBL-132 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 154 |
| Q-SBL-133 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 155 |
| Q-SBL-133 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 156 |
| Q-SBL-134 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 157 |
| Q-SBL-134 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 158 |
| Q-SBL-135 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 159 |
| Q-SBL-135 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP | 160 |
| | REAKVQWKVDNALQSGNSQESVTEQDSKDSTYSLSSTLTLSKADY EKHKVYACEVTHQGLSSPVTKSFNRGEC | |
| Q-SBL-136 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 161 |
| Q-SBL-136 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 162 |
| Q-SBL-137 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 163 |
| Q-SBL-137 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 164 |
| Q-SBL-138 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 165 |
| Q-SBL-138 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 166 |
| Q-SBL-139 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 167 |
| Q-SBL-139 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 168 |
| Q-SBL-140 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT | 169 |
| | TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | |
| Q-SBL-140 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 170 |
| Q-SBL-141 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVYTLPPCREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 171 |
| Q-SBL-141 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 172 |
| Q-SBL-142 LC (First arm light chain region) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTPREPQVCTLPPSREEMTKNQVSLWCLVKGF YPSDIAVEWESNGQPENNYKTTPPVLDSDGSFFLYSKLTVDKSRWQ QGNVFSCSVMHEALHNHYTQKSLSLSPGKGGGGSRTVAAPSVFIFP PSDEQLKSGTASVVCLLNNFYPREAKVQWKVDNALQSGNSQESVT EQDSKDSTYSLSSTLTLSKADYEKHKVYACEVTHQGLSSPVTKSFN RGES | 173 |
| Q-SBL-142 LC (Second arm light chain region) | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC | 174 |

### Example 2. Production of bispecific antibodies including CH3 domain variants

A vector plasmid containing the coding genes of the heavy chain region and light chain region (Q-SBL) of the first arm and a vector plasmid containing the coding genes of the heavy chain region and light chain region of the second arm were constructed. To express the second arm, genes encoding the heavy and light chains were inserted into one vector plasmid. The promoter used was CMV, and WPRE (woodchuck hepatitis virus post-transcriptional regulatory element) was inserted behind the coding gene to increase the expression level during transient expression.

A vector plasmid containing the coding gene of the first arm and a vector plasmid containing the coding gene of the second arm were co-transfected into ExpiCHO-S animal cells (Thermo Fisher, A29127). As such, the ratio of the vector plasmid for expressing the first arm to the vector plasmid for expressing the second arm was set to 1:1 (Q-SBL). For transfection, the ExpiFectamine CHO Transfection Kit (Thermo Fisher, A29130) was used according to the manufacturer's protocol. 13 days after transfection, to obtain a bispecific antibody, the supernatant was collected by centrifugation (10,000 rpm, 15 minutes), and residual cell debris was removed by filtration of the supernatant through a 0.22 µm filter paper.

### Example 3. Expression of bispecific antibodies including CH3 domain variants

Expression yields of bispecific antibodies including CH3 domain variants were measured on an Octet instrument [Table 4]. Using a Protein A biosensor (Sartorius 18-5010), a calibration curve was created using an IgG1 type standard substance, and the bispecific antibody concentration in the CHO cell culture supernatant (harvest cell culture fluid, HCCF) was determined using the calibration curve.

**[Table 4]**

| Antibody concentration in culture supernatant quantified by Octet analysis | | | |
|---|---|---|---|
| **Sample ID** | **HCCF titer (mg/L)** | **Sample ID** | **HCCF titer (mg/L)** |
| Q-SBL-100 | 338.8 | Q-SBL-122 | 208.5 |
| Q-SBL-101 | 297.2 | Q-SBL-123 | 270.7 |
| Q-SBL-102 | 176.6 | Q-SBL-124 | 371.3 |
| Q-SBL-103 | 210.6 | Q-SBL-125 | 422.5 |
| Q-SBL-104 | 298.7 | Q-SBL-126 | 361.0 |
| Q-SBL-105 | 376.6 | Q-SBL-127 | 168.0 |
| Q-SBL-106 | 287.6 | Q-SBL-128 | 165.7 |
| Q-SBL-107 | 197.8 | Q-SBL-129 | 95.8 |
| Q-SBL-108 | 383.9 | Q-SBL-130 | 152.5 |
| Q-SBL-109 | 231.2 | Q-SBL-131 | 117.9 |
| Q-SBL-110 | 182.4 | Q-SBL-132 | 105.1 |
| Q-SBL-111 | 228.9 | Q-SBL-133 | 137.6 |
| Q-SBL-112 | 247.4 | Q-SBL-134 | 78.6 |
| Q-SBL-113 | 198.2 | Q-SBL-135 | 184.2 |
| Q-SBL-114 | 231.1 | Q-SBL-136 | 164.2 |
| Q-SBL-115 | 148.3 | Q-SBL-137 | 174.6 |
| Q-SBL-116 | 197.4 | Q-SBL-138 | 129.3 |
| Q-SBL-117 | 238.2 | Q-SBL-139 | 146.4 |
| Q-SBL-118 | 124.9 | Q-SBL-140 | 140.4 |
| Q-SBL-119 | 129.9 | Q-SBL-141 | 112.5 |
| Q-SBL-120 | 265.6 | Q-SBL-142 | 151.0 |
| Q-SBL-121 | 284.5 | | |

### Example 4. Purification of bispecific antibodies including CH3 domain mutants

In order to study and perform various analyses on bispecific antibodies to which the CH3 domain mutation candidates of the present invention were applied, the culture fluid obtained from the expression of the candidates was subjected to primary bispecific antibody purification using a MabSelect Sure (Cytiva) column for Protein A affinity chromatography. The bispecific antibody substance obtained in the primary purification process step was identified by performing non-reducing SDS-PAGE using a 3-8% Tris-acetate gel and Tris-acetate buffer. Additionally, reducing SDS-PAGE using a 4-12% Bis-Tris gel and Bis-Tris buffer was performed to confirm the presence of fragments other than those derived from the bispecific antibody.

Specifically, after equilibrating the MabSelect Sure column with 50 mM Tris-HCl (pH 7.0) buffer (equilibration buffer), the bispecific antibody culture fluid including the mutant filtered through a 0.22 µm filter paper was loaded onto the column. Proteins not bound to the column were washed away using the equilibration buffer described above, and impurities nonspecifically bound to the column were removed sequentially using equilibration buffer containing 0.5 M sodium chloride and 20 mM Bis-Tris (pH 5.8) buffer. Afterwards, the bispecific antibody specifically bound to the column was eluted by flow of 0.2 M glycine-HCl (pH 3.2) buffer. The eluted sample was neutralized to pH 5.0 using a 1.0 M Tris solution and then filtered through a 0.22 µm filter paper.

The subsequent purification process was performed using a Capto SP (Cytiva) column for cation exchange chromatography, a type of ion exchange chromatography, and the details were as follows. After stabilizing the Capto SP column with 50 mM Bis-Tris (pH 6.0) buffer, a bispecific antibody sample titrated to pH 6.0 was applied, and impurities not bound to the column were washed with the same buffer. The bispecific antibody bound to the column was eluted using sodium chloride at concentrations ranging from 0.1 M to 1.0 M.

The final purification process involved hydrophobic interaction chromatography to remove high-molecular-weight (HMW) and low-molecular-weight (LMW) impurities from the bispecific antibody sample having undergone secondary purification. In this process, a butyl series Sepharose column was used, and the sample was prepared by buffer-exchanging the bispecific antibody preparation purified by ion exchange chromatography with high-concentration salt buffer to achieve a final salt concentration of 1.0 M to 1.5 M. After equilibrating the column with 50 mM Bis-Tris (pH 6.0) buffer having the same salt concentration as the applied sample, the prepared sample was loaded. The bound bispecific antibody was eluted in a gradient manner for 30 CV with 50 mM salt-free Bis-Tris (pH 6.0). The final purified product of the eluted bispecific antibody was concentrated to a concentration of 1 to 2 mg/ml using a 50 kDa molecular-weight cut-off ultrafiltration tube and then buffer-exchanged with appropriate buffer depending on the analysis conditions.

### Example 5. Purity analysis using size-exclusion high-pressure chromatography (SEC-HPLC)

Size-exclusion high-pressure chromatography was performed to evaluate the aggregation and heterodimer purity of the bispecific antibody candidates. About 50 µg of the purified product that had passed through the Protein A resin was passed through a TSKGel G3000SWXL (Tosoh) column at a flow rate of 0.5 ml/min with a mobile phase containing 250 mM potassium phosphate and 200 mM potassium chloride, pH 7.2 using an Alliance 2695 HPLC system with a 2489 UV/Vis detector from Waters, and UV absorbance was measured at 280 nm. System suitability was confirmed using Gel Filtration Standard (151-1901) from Bio-Rad, and after analysis, the data were integrated using the manufacturer's Empower 3.0 software to calculate purity as a percentage of peak area. The results of SEC-HPLC analysis are shown in [Table 5] below.

**[Table 5]**

| Purity analysis of rPA-purified products of bispecific antibody candidates using SEC-HPLC | | | |
|---|---|---|---|
| **Sample ID** | **SEC-HPLC (rPA purification, %)** | **Sample ID** | **SEC-HPLC (rPA purification, %)** |
| Q-SBL-100 | 60.9 | Q-SBL-122 | 72.64 |
| Q-SBL-101 | 68.8 | Q-SBL-123 | 72.94 |
| Q-SBL-102 | 66.2 | Q-SBL-124 | 67.53 |
| Q-SBL-103 | 63.5 | Q-SBL-125 | 70.19 |
| Q-SBL-104 | 45.47 | Q-SBL-126 | 65.28 |
| Q-SBL-105 | 69.38 | Q-SBL-127 | 84.56 |
| Q-SBL-106 | 32.83 | Q-SBL-128 | 58.31 |
| Q-SBL-107 | 48.91 | Q-SBL-129 | 69.63 |
| Q-SBL-108 | 82.50 | Q-SBL-130 | 62.09 |
| Q-SBL-109 | 75.70 | Q-SBL-131 | 52.64 |
| Q-SBL-110 | 87.30 | Q-SBL-132 | 78.34 |
| Q-SBL-111 | 80.20 | Q-SBL-133 | 12.21 |
| Q-SBL-112 | 83.3 | Q-SBL-134 | 12.64 |
| Q-SBL-113 | 74.6 | Q-SBL-135 | 81.29 |
| Q-SBL-114 | 83.2 | Q-SBL-136 | 82.02 |
| Q-SBL-115 | 80.3 | Q-SBL-137 | 81.52 |
| Q-SBL-116 | 50.34 | Q-SBL-138 | 49.47 |
| Q-SBL-117 | 51.83 | Q-SBL-139 | 50.44 |
| Q-SBL-118 | 50.23 | Q-SBL-140 | 48.10 |
| Q-SBL-119 | 64.82 | Q-SBL-141 | 7.17 |
| Q-SBL-120 | N.D. | Q-SBL-142 | 80.86 |
| Q-SBL-121 | 57.05 | N/A | N/A |

### Example 6. CE-SDS analysis

For purity analysis of bispecific antibody candidates under non-reducing or reducing conditions, sodium dodecyl sulfate capillary electrophoresis (CE-SDS) was performed using a PA 800 Plus system (Sciex) and the IgG Purity and Heterogeneity Assay Kit (Sciex). For sample analysis after purification using Protein A resin, 50 µl of protein was used at a maximum concentration of 2 mg/ml. For analysis, each sample was mixed with 5 µl of 250 mM iodoacetamide or 14.2 M 2-mercaptoethanol, 2 µl of internal standard, and 45 µl of sodium dodecyl sulfate sample buffer, and then heated at 70°C for 10 minutes (CE-SDS Method 1). Alternatively, 20 µl of protein was used at a maximum concentration of 3.5 mg/ml for sample analysis after purification using Protein A resin. For analysis, each sample was mixed with 5 µl of 250 mM iodoacetamide or 14.2 M 2-mercaptoethanol, 2 µl of internal standard, and 75 µl of sodium dodecyl sulfate sample buffer, and then heated at 70°C for 10 minutes (CE-SDS Method 2).

After completion of analysis, the data were analyzed in 32 Karat^{™} software version 10.3 provided by the manufacturer.

[Table 6] below shows the results of CE-SDS analysis under non-reducing conditions after purification using Protein A resin. After purification of the bispecific antibody candidates using Protein A resin, the purity of the heterodimer was determined by sodium dodecyl sulfate capillary electrophoresis (CE-SDS).

**[Table 6]**

| Purity of bispecific antibodies under non-reducing conditions | | | |
|---|---|---|---|
| **Sample ID** | **CE-SDS (rPA purification, Non-reduced, %)** | **Sample ID** | **CE-SDS (rPA purification, Non-reduced, %)** |
| Q-SBL-100 | 50.91 | Q-SBL-122 | 74.58 |
| Q-SBL-101 | 57.97 | Q-SBL-123 | 81.86 |
| Q-SBL-102 | 52.96 | Q-SBL-124 | 80.16 |
| Q-SBL-103 | 42.56 | Q-SBL-125 | 83.09 |
| Q-SBL-104 | 20.30 | Q-SBL-126 | 80.13 |
| Q-SBL-105 | 54.64 | Q-SBL-127 | 84.56 |
| Q-SBL-106 | 38.42 | Q-SBL-128 | 60.98 |
| Q-SBL-107 | 36.41 | Q-SBL-129 | 73.28 |
| Q-SBL-108 | 83.06 | Q-SBL-130 | 67.22 |
| Q-SBL-109 | 77.68 | Q-SBL-131 | 56.16 |
| Q-SBL-110 | 83.07 | Q-SBL-132 | 13.06 |
| Q-SBL-111 | 86.49 | Q-SBL-133 | 11.33 |
| Q-SBL-112 | 73.69 | Q-SBL-134 | 15.01 |
| Q-SBL-113 | 70.55 | Q-SBL-135 | 89.18 |
| Q-SBL-114 | 87.94 | Q-SBL-136 | 90.63 |
| Q-SBL-115 | 61.30 | Q-SBL-137 | 86.14 |
| Q-SBL-116 | 39.56 | Q-SBL-138 | 45.24 |
| Q-SBL-117 | 45.90 | Q-SBL-139 | 45.87 |
| Q-SBL-118 | 51.38 | Q-SBL-140 | 43.96 |
| Q-SBL-119 | 56.02 | Q-SBL-141 | 11.27 |
| Q-SBL-120 | 85.92 | Q-SBL-142 | 87.96 |
| Q-SBL-121 | 89.26 | | |

### Example 7. Single antigen binding ELISA

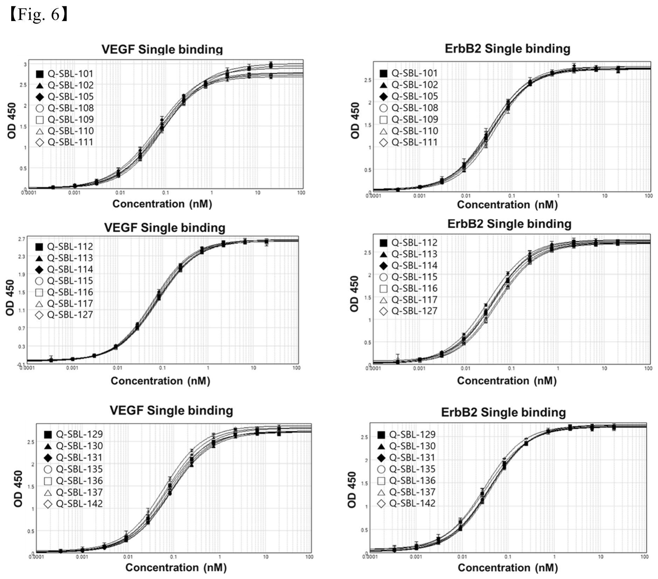

Since the bispecific antibody according to the present invention targets multiple antigens, both single antigen binding and dual antigen binding ELISAs were conducted, and the specific procedure for single antigen binding ELISA is as follows. Depending on the target to be identified, rhErbB2-his tag and rhVEGF 165 recombinant protein (R&D Systems) were applied onto a 96-well immunoassay ELISA plate using 1x PBS, pH 7.4. As such, the coating solutions were prepared at concentrations of 1 µg/ml for rhErbB2-his tag and 0.5 µg/ml for rhVEGF, and 100 µl thereof was added to each well. The plate was incubated at 37°C for 1 hour to allow coating with rhErbB2-his tag and rhVEGF 165, and then washed five times with 0.05% PBS-T. To prevent nonspecific binding, each well was treated with 200 µl of 2% BSA for blocking, incubated at 37°C for 30 minutes, and then washed five times with 0.05% PBS-T. The bispecific antibody according to the present invention was serially diluted (from 20 to 0.0001 nM) in 2% BSA, and 100 µl thereof was added to each well of the recombinant protein-coated plate, and the plate was incubated at 37°C for 1 hour and then washed five times with 0.05% PBS-T. Next, the HRP-conjugated secondary antibody was diluted (anti-hIgG FC-HRP 1:10,000) in PBS containing 2% BSA, 100 µl thereof was added to each well, and the plate was incubated at 37°C for 30 minutes and then washed five times with 0.05% PBS-T. 100 µl of TMB (Bio-Rad), which reacts with HRP of the secondary antibody to develop color, was added to each well, followed by color development at room temperature for 5 minutes, after which 100 µl of 1 M H₂SO₄ was added to each well to terminate color development, and then absorbance was measured at a wavelength of 450 nm using a SpectraMax ABS Plus (Molecular Devices). Therefore, the EC₅₀ values for single antigen binding of the CH3 engineering group including the ErbB2 and VEGF binding regions are shown in [Table 7] below. [FIG. 6] shows a graph of 4-parameter fitting of binding affinity analysis for each single antigen of ErbB2 and VEGF of the candidate group.

**[Table 7]**

| EC₅₀ values for single antigen binding ELISA of bispecific antibodies including ErbB2 and VEGF binding regions | | | | |
|---|---|---|---|---|
| **Sample ID** | **ErbB2** | | **VEGF** | |
| | **EC₅₀ (nM)** | **R²** | **EC₅₀ (nM)** | **R²** |
| Q-SBL-100 | 0.057 | 1.000 | 0.100 | 0.999 |
| Q-SBL-101 | 0.037 | 1.000 | 0.088 | 1.000 |
| Q-SBL-102 | 0.038 | 1.000 | 0.078 | 1.000 |
| Q-SBL-103 | 0.061 | 0.999 | 0.096 | 0.999 |
| Q-SBL-105 | 0.033 | 1.000 | 0.061 | 1.000 |
| Q-SBL-108 | 0.032 | 1.000 | 0.066 | 1.000 |
| Q-SBL-109 | 0.039 | 1.000 | 0.080 | 1.000 |
| Q-SBL-110 | 0.035 | 1.000 | 0.069 | 1.000 |
| Q-SBL-111 | 0.042 | 1.000 | 0.082 | 1.000 |
| Q-SBL-112 | 0.039 | 1.000 | 0.072 | 1.000 |
| Q-SBL-113 | 0.036 | 1.000 | 0.076 | 1.000 |
| Q-SBL-114 | 0.031 | 1.000 | 0.062 | 1.000 |
| Q-SBL-115 | 0.038 | 1.000 | 0.064 | 1.000 |
| Q-SBL-116 | 0.049 | 1.000 | 0.080 | 1.000 |
| Q-SBL-117 | 0.045 | 1.000 | 0.076 | 1.000 |
| Q-SBL-127 | 0.040 | 1.000 | 0.072 | 1.000 |
| Q-SBL-129 | 0.032 | 1.000 | 0.069 | 1.000 |
| Q-SBL-130 | 0.039 | 1.000 | 0.084 | 1.000 |
| Q-SBL-131 | 0.039 | 1.000 | 0.087 | 1.000 |
| Q-SBL-135 | 0.039 | 1.000 | 0.084 | 1.000 |
| Q-SBL-136 | 0.042 | 1.000 | 0.081 | 1.000 |
| Q-SBL-137 | 0.039 | 1.000 | 0.069 | 1.000 |
| Q-SBL-142 | 0.031 | 1.000 | 0.060 | 1.000 |

### Example 8. Dual antigen binding ELISA

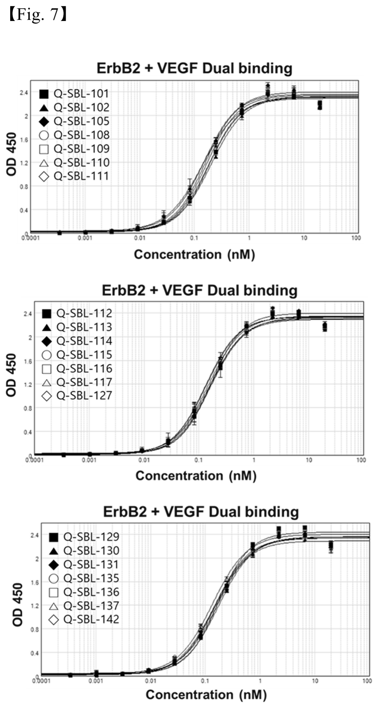

The specific procedure for dual antigen binding ELISA is as follows. Depending on the target to be identified, rhVEGF 165 recombinant protein (R&D Systems) was applied onto a 96-well immunoassay ELISA plate using 1x PBS, pH 7.4 at a coating concentration of 1 µg/ml (100 µl per well), and the plate was incubated at 4°C for 18 hours to allow coating with rhVEGF 165 and then washed five times with 0.05% PBS-T. To prevent nonspecific binding, each well was treated with 200 µl of 2% BSA for blocking, incubated at 37°C for 2 hours, and then washed five times with 0.05% PBS-T. The bispecific antibody serially diluted in 2% BSA at a 1/3 ratio from a maximum concentration of 100 nM to 0.0016 nM and rhErbB2-his (R&D Systems) diluted to 1 µg/ml were mixed 1:1 and incubated at 37°C for 2 hours. After washing the microplate five times with 0.05% PBS-T, 100 µl of the mixed antibody and rh ErbB2-his sample was added to each well, and the plate was incubated at 37°C for 2 hours and then washed five times with 0.05% PBS-T. Afterwards, the HRP-conjugated anti-secondary antibody was diluted (anti-his tag-HRP 1:10,000) in PBS containing 2% BSA, 100 µl thereof was added to each well, and the plate was incubated at 37°C for 1 hour and then washed five times with 0.05% PBS-T. 100 µl of TMB (Bio-Rad) was added to each well, followed by color development at room temperature for 5 minutes, after which 100 µl of 1 M H₂SO₄ was added to each well to terminate color development. Absorbance was measured at a wavelength of 450 nm using a SpectraMax ABS Plus (Molecular Devices). Therefore, the EC₅₀ values for dual antigen binding of the CH3 engineering group including ErbB2 and VEGF binding regions are shown in [Table 8] below. [FIG. 7] shows a graph of 4-parameter fitting of dual antigen binding affinity analysis of ErbB2 and VEGF of the candidate group.

**[Table 8]**

| EC₅₀ values for dual antigen binding ELISA of bispecific antibodies including ErbB2 and VEGF binding regions | | |
|---|---|---|
| **Sample ID** | **VEGF+ ErbB2** | |
| | **EC₅₀ (nM)** | **R²** |
| Q-SBL-100 | 0.153 | 0.995 |
| Q-SBL-101 | 0.183 | 0.998 |
| Q-SBL-102 | 0.202 | 0.996 |
| Q-SBL-103 | 0.151 | 0.998 |
| Q-SBL-105 | 0.150 | 0.994 |
| Q-SBL-108 | 0.157 | 0.994 |
| Q-SBL-109 | 0.186 | 0.995 |
| Q-SBL-110 | 0.168 | 0.997 |
| Q-SBL-111 | 0.140 | 0.995 |
| Q-SBL-112 | 0.149 | 0.996 |
| Q-SBL-113 | 0.154 | 0.995 |
| Q-SBL-114 | 0.165 | 0.995 |
| Q-SBL-115 | 0.132 | 0.997 |
| Q-SBL-116 | 0.162 | 0.996 |
| Q-SBL-117 | 0.163 | 0.997 |
| Q-SBL-127 | 0.136 | 0.996 |
| Q-SBL-129 | 0.170 | 0.995 |
| Q-SBL-130 | 0.174 | 0.996 |
| Q-SBL-131 | 0.165 | 0.996 |
| Q-SBL-135 | 0.134 | 0.994 |
| Q-SBL-136 | 0.145 | 0.996 |
| Q-SBL-137 | 0.147 | 0.997 |
| Q-SBL-142 | 0.147 | 0.994 |

### Example 9. Fluorescence-activated cell sorting (FACS)

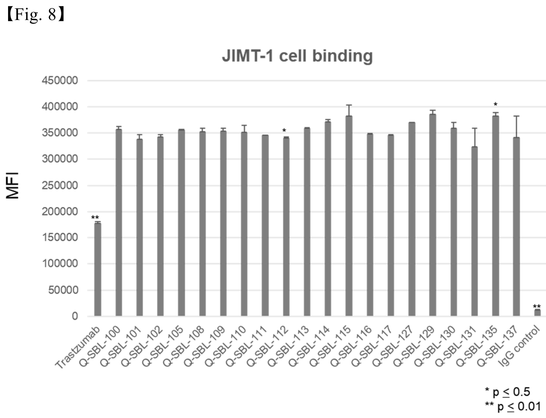

Fluorescence-activated cell sorting was performed to determine whether antigen binding ability at the cellular level was maintained when introducing the CH3 domain variant according to the present invention into a bispecific antibody. The specific procedure for fluorescence-activated cell sorting is as follows. JIMT-1 cells expressing HER2, the target to be identified, were subcultured in DMEM containing 1% penicillin-streptomycin (Gibco, 15140-122) and 10% fetal bovine serum (Gibco, 16140071) at 37°C in a 5% CO₂ incubator. As such, the cell density in a 75-T flask was maintained below 80% confluence. After JIMT-1 cells were prepared in a 96-well plate at a density of 2x10⁵ cells/well, the bispecific antibody according to the present invention was prepared at 100 nM using FACS staining buffer (Invitrogen, 00-4222-26), and 100 µl thereof was added to each well and allowed to react with the cells at 4°C for 1 hour. The supernatant was removed by centrifugation (1,000 rpm, 5 minutes), and the antibody-bound cells were washed three times with 100 µl of FACS buffer to prevent nonspecific binding. The FITC-conjugated secondary antibody was diluted in FACS buffer (Abcam, ab239228, 1:40) and 100 µl thereof was added to each well and allowed to react with the cells at 4°C for 30 minutes, and the same washing process as above was repeated three times. The antibody-bound cells were analyzed using a fluorescence-activated cell sorter (Satorius, iQue^{®} 3), and fluorescence emission signals were detected through a FITC filter with excitation at 498 nm and emission collected in the range of 475 to 650 nm. The results are depicted in the figure, and the results quantified by the mean fluorescence intensity (MFI) are shown in Table 9 and FIG. 8.

**[Table 9]**

| Quantitative comparison of MFI values for binding strength between bispecific antibody and ErbB2 expressed on surface of JIMT-1 cells | |
|---|---|
| **Sample ID** | **MFI** |
| Trastuzumab | 177257.5±4263.1 |
| Q-SBL-100 | 357021.5±5021.2 |
| Q-SBL-101 | 337523±9727.0 |
| Q-SBL-102 | 342669.5±4305.6 |
| Q-SBL-105 | 355521.5±1276.3 |
| Q-SBL-108 | 352517±6662.4 |
| Q-SBL-109 | 353999.5±4780.7 |
| Q-SBL-110 | 351091.5±13407.5 |
| Q-SBL-111 | 345416.5±480.1 |
| Q-SBL-112 | 340176.5±2211.1 |
| Q-SBL-113 | 358573.5+1699.2 |
| Q-SBL-114 | 371240±3872.1 |
| Q-SBL-115 | 382186±20719.6 |
| Q-SBL-116 | 347727+1888.0 |
| Q-SBL-117 | 346295±478.0 |
| Q-SBL-127 | 369969±281.4 |
| Q-SBL-129 | 38504718414.6 |
| Q-SBL-130 | 358608.5±11025.9 |
| Q-SBL-131 | 323844.5±35713.8 |
| Q-SBL-135 | 382750±6001.9 |
| Q-SBL-137 | 341426±40784.5 |
| IgG control | 12479±154.1 |

### Example 9. Binding kinetics analysis of FcγRIIa, FcyRIIIa, and Clq

The Fc region (hinge-CH2) of IgG1 interacts with the Fcγ receptor (FcγR) and complement protein (Clq, complement component 1q) to induce immune effector function. In order to determine whether the binding ability to FcγRIIa, FcγRIIIa, and Clq was maintained when the CH3 domain variant according to the present invention was introduced into the IgG1 framework, binding kinetics analysis was performed using the Octet^{®} HTX system. The specific procedure for binding kinetics analysis was as follows.

FcγRIIa: After baseline adjustment for 60 seconds with analysis buffer (1x PBS containing 0.25% sodium azide + 0.5% BSA + 0.1% Tween-20) on the FAB2G Biosensor, 100 nM of the bispecific antibody was immobilized for 300 seconds, baseline adjustment was again performed for 60 seconds, and the serially diluted FcγRIIa samples (4000, 2000, 1000, 500, 250, 125, 62.5, and 0 nM) were allowed to associate and dissociate for 60 seconds each. K_{D} values were calculated using Data Analysis 12.0 software.

**[Table 10]**

| K_{D} values for FcγRIIa binding of bispecific antibodies | | |
|---|---|---|
| **Sample ID** | **K_{D} (JIM)** | **Full R²** |
| Q-SBL-100 | 1.19+0.18 | |
| Q-SBL-101 | 2.46±0.25 | |
| Q-SBL-102 | 1.70±0.07 | |
| Q-SBL-103 | 1.13±0.12 | |
| Q-SBL-104 | 1.87±0.17 | |
| Q-SBL-105 | 1.91±0.09 | |
| Q-SBL-108 | 2.37±0.18 | |
| Q-SBL-109 | 2.20±0.00 | >0.99 |
| Q-SBL-110 | 2.29±0.06 | |
| Q-SBL-111 | 2.04±0.08 | |
| Q-SBL-112 | 2.02±0.17 | |
| Q-SBL-113 | 2.14±0.08 | |
| Q-SBL-114 | 1.86±0.09 | |
| Q-SBL-115 | 1.97±0.08 | |
| Q-SBL-116 | 2.34±0.04 | |
| Q-SBL-117 | 2.55±0.14 | |
| Q-SBL-127 | 1.63+0.04 | |
| Q-SBL-129 | 1.67±0.10 | |
| Q-SBL-130 | 1.72±0.00 | |
| Q-SBL-131 | 1.62±0.08 | |
| Q-SBL-135 | 1.38±0.05 | |
| Q-SBL-136 | 1.97±0.06 | |
| Q-SBL-137 | 2.12±0.03 | |
| Q-SBL-142 | 1.79+0.12 | |

FcγRIIIa: After baseline adjustment for 60 seconds with 1x kinetics buffer on the FAB2G Biosensor, 100 nM of the bispecific antibody was immobilized for 300 seconds, baseline adjustment was again performed for 60 seconds, and the serially diluted FcγRIIIa samples (2000, 1000, 500, 250, 125, 62.5, 31.25, and 0 nM) were allowed to associate and dissociate for 60 seconds each.

**[Table 11]**

| K_{D} values for FcγRIIIa binding of bispecific antibodies | | |
|---|---|---|
| **Sample ID** | **K_{D} (JIM)** | **Full R²** |
| Q-SBL-100 | 0.25±0.70 | |
| Q-SBL-101 | 0.50±0.04 | >0.99 |
| Q-SBL-102 | 0.42±0.03 | |
| Q-SBL-103 | 0.22±0.10 | |
| Q-SBL-104 | ND | ND |
| Q-SBL-105 | 0.43±0.02 | |
| Q-SBL-108 | 0.34±0.00 | |
| Q-SBL-109 | 0.34±0.04 | |
| Q-SBL-110 | 0.57±0.05 | >0.99 |
| Q-SBL-111 | 0.49±0.02 | |
| Q-SBL-112 | 0.60±0.02 | |
| Q-SBL-113 | 0.60±0.04 | |
| Q-SBL-114 | 0.23±0.00 | |
| Q-SBL-115 | 0.29±0.00 | |
| Q-SBL-116 | 0.37±0.03 | |
| Q-SBL-117 | 0.36±0.00 | |
| Q-SBL-127 | 0.23±0.00 | |
| Q-SBL-129 | 0.29±0.03 | |
| Q-SBL-130 | 0.29±0.00 | |
| Q-SBL-131 | 0.33±0.02 | |
| Q-SBL-135 | 0.30±0.06 | |
| Q-SBL-136 | 0.23±0.01 | |
| Q-SBL-137 | 0.38±0.02 | |
| Q-SBL-142 | 0.27±0.03 | |

Clq: After baseline adjustment for 60 seconds with analysis buffer (1x PBS containing 200 mM NaCl + 0.3% BSA + 0.05% Tween-20) on the FAB2G Biosensor, 100 nM of the bispecific antibody was immobilized for 300 seconds, baseline adjustment was again performed for 60 seconds, and the serially diluted C1q samples (200, 100, 50, 25, 12.5, 6.25, 3.125, and 0 nM) were allowed to associate for 30 seconds and dissociate for 60 seconds.

**[Table 12]**

| K_{D} values for C1q binding of bispecific antibodies | | |
|---|---|---|
| **Sample ID** | **K_{D} (JIM)** | **Full R²** |
| Q-SBL-100 | 0.038±0.001 | >0.99 |
| Q-SBL-101 | 0.036±0.001 | |
| Q-SBL-102 | 0.040±0.007 | |
| Q-SBL-103 | 0.040±0.001 | |
| Q-SBL-105 | ND | ND |
| Q-SBL-108 | 0.028±0.003 | >0.99 |
| Q-SBL-109 | 0.028±0.002 | |
| Q-SBL-110 | 0.029±0.001 | |
| Q-SBL-111 | 0.030±0.003 | |
| Q-SBL-112 | 0.032±0.000 | |
| Q-SBL-113 | 0.036±0.002 | |
| Q-SBL-114 | 0.018±0.000 | |
| Q-SBL-115 | 0.018±0.000 | |
| Q-SBL-116 | 0.033±0.001 | |
| Q-SBL-117 | 0.040±0.003 | |
| Q-SBL-127 | 0.018±0.001 | |
| Q-SBL-129 | 0.016±0.000 | |
| Q-SBL-130 | 0.017±0.001 | |
| Q-SBL-131 | 0.016±0.002 | |
| Q-SBL-137 | 0.024±0.002 | |
| Q-SBL-142 | 0.023±0.001 | |

### Example 10. FcRn binding kinetics analysis

The Fc region (CH2-CH3) of IgG1 plays an important role in enhancing the half-life of antibodies by interacting with FcRn and mediating recycling of antibodies. In order to determine whether the binding ability to FcRn was maintained when the CH3 domain variant according to the present invention was introduced into a bispecific antibody, binding kinetics analysis was performed using the Octet^{®} HTX system. The specific procedure for binding kinetics analysis was as follows. After baseline adjustment for 60 seconds with 1x kinetics buffer on the FAB2G Biosensor, the bispecific antibody diluted to 100 nM was immobilized for 300 seconds, baseline adjustment was again performed for 180 seconds with analysis buffer at pH 6.0, and the serially diluted FcRn samples (1600, 800, 400, 200, 100, 50, 25, and 0 nM) were allowed to associate for 30 seconds and dissociate for 60 seconds. K_{D} values were calculated using Data Analysis 12.0 software.

**[Table 13]**

| K_{D} values for FcRn binding of bispecific antibodies | | |
|---|---|---|
| **Sample ID** | **K_{D} (µM)** | **Full R²** |
| Q-SBL-101 | 0.73±0.01 | |
| Q-SBL-102 | 0.71±0.02 | |
| Q-SBL-104 | 0.68±0.00 | >0.99 |
| Q-SBL-105 | 0.71±0.00 | |
| Q-SBL-106 | 0.68±0.06 | |
| Q-SBL-107 | ND | ND |
| Q-SBL-108 | 0.64±0.02 | |
| Q-SBL-109 | 0.64±0.06 | |
| Q-SBL-110 | 0.61±0.06 | |
| Q-SBL-111 | 0.69±0.01 | >0.99 |
| Q-SBL-112 | 0.72±0.01 | |
| Q-SBL-113 | 0.69±0.01 | |
| Q-SBL-115 | 0.69±0.01 | |
| Q-SBL-116 | 0.65±0.01 | |
| Q-SBL-117 | ND | ND |
| Q-SBL-127 | 0.75±0.01 | |
| Q-SBL-129 | 0.70±0.04 | |
| Q-SBL-130 | 0.74±0.03 | >0.99 |
| Q-SBL-131 | 0.67±0.01 | |
| Q-SBL-135 | 0.65±0.02 | |
| Q-SBL-136 | 0.62±0.01 | |
| Q-SBL-137 | 0.58±0.02 | |
| Q-SBL-142 | 0.57±0.01 | |

### [Industrial Applicability]

The present invention provides a heterodimer including CH3 domains with an increased yield of heterodimer formation.

In addition, according to the present invention, by introducing asymmetric amino acid mutations into the CH3 domain of the heavy chain constant region, the formation of homodimers during the production of bispecific antibodies can be minimized, and the yield of heterodimer formation can be increased.

In addition, the bispecific antibody produced using the CH3 domain variant of the heavy chain constant region according to the present invention is capable of retaining the target antigen binding ability of the original wild-type antibody as well as the intrinsic functions of the heavy chain constant region (Fc: Hinge-CH2-CH3), including binding to the Fcy receptor (FcγR), complement protein (C1q, complement component 1q), and FcRn (neonental Fc receptor), thereby preserving antibody-dependent immune effector function and blood half-life.

Moreover, the present invention provides a bispecific antibody format that alleviates nonspecific binding of heavy and light chains or homodimer formation in conventional bispecific antibody formats that simultaneously bind to the first antigen and the second antigen. By introducing asymmetric amino acid mutations into the CH3 domain of the heavy chain constant region and introducing other asymmetric amino acid mutations into the CH3 domain of the antibody variable region additionally applied to the proprietary bispecific antibody format, the formation of homodimers during the production of bispecific antibodies can be minimized, and the yield of heterodimer formation can be increased.

Having described certain parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Sequence List Free Text]

An electronic file is attached.

## Claims

1. A heterodimer comprising a first heavy chain CH3 domain and a second heavy chain CH3 domain,
wherein the first heavy chain CH3 domain or the second heavy chain CH3 domain comprises at least one amino acid substitution (according to an EU index) at a position selected from the group consisting of:
(1) substitution of an amino acid at position Q347, E356, or E357 with K or R;
(2) substitution of an amino acid at position D399 with W, F, Y, or H;
(3) substitution of an amino acid at position K360, K370, or K439 with Q, N, E, or D; and
(3) substitution of an amino acid at position K409 with A, V, T, or S, and
the first heavy chain CH3 domain and the second heavy chain CH3 domain bind to form a dimer.

2. The heterodimer according to claim 1, wherein the amino acid at position Q347, E356, or E357 in the first heavy chain CH3 domain is substituted with K or R, or the amino acid at position D399 is substituted with W, F, Y, or H.

3. The heterodimer according to claim 1, wherein the amino acid at position K360, K370, or K439 in the second heavy chain CH3 domain is substituted with Q, N, E, or D, or the amino acid at position K409 is substituted with A, V, T, or S.

4. The heterodimer according to claim 1, comprising Q347K or Q347R in the first heavy chain CH3 domain, and K360Q, K360N, K360E, or K360D in the second heavy chain CH3 domain.

5. The heterodimer according to claim 1, comprising E357K or E357R in the first heavy chain CH3 domain, and K370Q, K370N, K370E, or K370D in the second heavy chain CH3 domain.

6. The heterodimer according to claim 1, comprising E356K or E356R in the first heavy chain CH3 domain, and K439Q, K439N, K439E, or K439D in the second heavy chain CH3 domain.

7. The heterodimer according to claim 1, comprising D399W, D399F, D399Y, or D399H in the first heavy chain CH3 domain, and K409A, K409T, K409V, or K409S in the second heavy chain CH3 domain.

8. The heterodimer according to claim 1, further comprising at least one selected from the group consisting of Y349C, S354C, T366S, T366W, L368A, and Y407V in the first heavy chain CH3 domain or the second heavy chain CH3 domain.

9. A fusion protein comprising the heterodimer according to any one of claims 1 to 8.

10. A bispecific antibody comprising a first arm that binds to a first antigen comprising VH1-CHa-Fc1 and VL1-CLb; and a second arm that binds to a second antigen comprising VH2-CH1-Fc2 and VL2-CL,
wherein the VH1 and VH2 are heavy chain variable regions comprising identical or different antigen binding regions,
the VL1 and VL2 are light chain variable regions comprising identical or different antigen binding regions,
the CHa comprises an IgG heavy chain constant region or an IgD heavy chain constant region CH1, and an IgG heavy chain constant region CH2 or CH3,
the CLb comprises at least one selected from the group consisting of CL1 comprising an IgG light chain constant region λ or κ, and IgG heavy chain constant regions CH1, CH2, and CH3,
the CH1 is an IgG heavy chain constant region CH1, and the CL is an IgG light chain constant region CL,
Fc1 of the first arm and Fc2 of the second arm bind to form a heavy chain constant region dimer, and
the bispecific antibody comprises a heterodimer comprising a first heavy chain CH3 domain and a second heavy chain CH3 domain, wherein CH3 of the CHa, Fc1 of the first arm, or Fc2 of the second arm comprises at least one amino acid substitution (according to an EU index) at a position selected from the group consisting of:
(1) substitution of an amino acid at position Q347, E356, or E357 with K or R;
(2) substitution of an amino acid at position D399 with W, F, Y, or H;
(3) substitution of an amino acid at position K360, K370, or K439 with Q, N, E, or D; and
(3) substitution of an amino acid at position K409 with A, V, T, or S.

11. The bispecific antibody according to claim 10, wherein the amino acid at position Q347, E356, or E357 in the first heavy chain CH3 domain is substituted with K or R, or the amino acid at position D399 is substituted with W, F, Y, or H.

12. The bispecific antibody according to claim 10, wherein the amino acid at position K360, K370, or K439 in the second heavy chain CH3 domain is substituted with Q, N, E, or D, or the amino acid at position K409 is substituted with A, V, T, or S.

13. The bispecific antibody according to claim 10, comprising Q347K or Q347R in the first heavy chain CH3 domain, and K360Q, K360N, K360E, or K360D in the second heavy chain CH3 domain.

14. The bispecific antibody according to claim 10, comprising E357K or E357R in the first heavy chain CH3 domain, and K370Q, K370N, K370E, or K370D in the second heavy chain CH3 domain.

15. The bispecific antibody according to claim 10, comprising E356K or E356R in the first heavy chain CH3 domain, and K439Q, K439N, K439E, or K439D in the second heavy chain CH3 domain.

16. The bispecific antibody according to claim 10, comprising D399W, D399F, D399Y, or D399H in the first heavy chain CH3 domain, and K409A, K409T, K409V, or K409S in the second heavy chain CH3 domain.

17. The bispecific antibody according to claim 10, further comprising at least one selected from the group consisting of Y349C, S354C, T366S, T366W, L368A, and Y407V in the first heavy chain CH3 domain or the second heavy chain CH3 domain.

18. The bispecific antibody according to claim 10, wherein CH3 and CH1 of the CHa or CH3 and CL1 of the CLb are connected by a linker.

19. The bispecific antibody according to claim 18, wherein the linker comprises 5 to 10 amino acid residues.

20. The bispecific antibody according to claim 18, wherein the first arm and the second arm are connected via a hinge.
